# EUROPEAN PATENT SPECIFICATION

(11) **EP 2 069 500 B1**
(45) Date of publication and mention of the grant of the patent: **24.09.2014**
(21) Application number: 07820947.5
(22) Date of filing: 04.10.2007
(51) Int. Cl.: C12N 15/11, C12N 15/87, A61K 31/713, C07J 41/00

(54) **COMPOSITIONS COMPRISING A SIRNA AND LIPIDIC 4,5-DISUBSTITUTED 2-DEOXYSTREPTAMINE RING AMINOGLYCOSIDE DERIVATIVES AND USES THEREOF**
ZUSAMMENSETZUNGEN MIT EINER SIRNA UND LIPIDISCHEN 4,5-DISUBSTITUIERTEN 2-DESOXYSTREPTAMINRING-AMINOGLYKOSID-DERIVATEN UND VERWENDUNGEN DAVON
COMPOSITIONS INCLUANT UN siARN ET DES DÉRIVÉS LIPIDIQUES D'AMINOGLYCOSIDE À NOYAU 2-DÉSOXYSTREPTAMINE 4,5-DISUBSTITUÉE ET LEURS UTILISATIONS

(30) Priority: 04.10.2006 US 849000 P
(43) Date of publication of application: 17.06.2009
(73) Proprietor: CENTRE NATIONAL DE LA RECHERCHE SCIENTIFIQUE (CNRS), 75016 Paris (FR); INSTITUT NATIONAL DE LA SANTE ET DE LA RECHERCHE MEDICALE (INSERM), 75013 Paris (FR)
(72) Inventor: LEHN, Jean-Marie, 67000 Strasbourg (FR); VIGNERON, Jean-Pierre, 91790 Boissy Sous Saint Yon (FR); LEHN, Pierre, 29200 Brest (FR); PITARD, Bruno, 44400 Reze (FR)
(74) Representative: Regimbeau
(86) International application number: PCT/EP2007/060571
(87) International publication number: WO 2008/040792

(56) References cited:
- WO-A-00/40692
- WO-A-03/018603
- WO-A-2005/014837
- SAINLOS MATTHIEU ET AL: "Kanamycin A-derived cationic lipids as vectors for gene transfection." CHEMBIOCHEM : A EUROPEAN JOURNAL OF CHEMICAL BIOLOGY, vol. 6, no. 6, June 2005 (2005-06), pages 1023-1033, XP002479876 ISSN: 1439-4227 cited in the application
- DESIGAUX LEA ET AL: "Self-assembled lamellar complexes of siRNA with lipidic aminoglycoside derivatives promote efficient siRNA delivery and interference" PROCEEDINGS OF THE NATIONAL ACADEMY OF SCIENCES OF THE UNITED STATES OF AMERICA, vol. 104, no. 42, October 2007 (2007-10), pages 16534-16539, XP002479877 ISSN: 0027-8424
- VANDERBYL SANDRA ET AL: "A flow cytometry technique for measuring chromosome-mediated gene transfer", CYTOMETRY, vol. 44, no. 2, 1 June 2001 (2001-06-01), pages 100-105, ISSN: 0196-4763
- MARTIN B ET AL: "The design of cationic lipids for gene delivery", CURRENT PHARMACEUTICAL DESIGN, BENTHAM SCIENCE PUBLISHERS, NL, vol. 11, no. 3, 1 January 2005 (2005-01-01), pages 375-394, XP002493549, ISSN: 1381-6128, DOI: 10.2174/1381612053382133
- HIRKO AARON ET AL: "Cationic lipid vectors for plasmid DNA delivery.", CURRENT MEDICINAL CHEMISTRY, vol. 10, no. 14, July 2003 (2003-07), pages 1185-1193, ISSN: 0929-8673
- SCHIFFELERS R M ET AL: "Effects of treatment with small interfering RNA on joint inflammation in mice with collagen-induced arthritis", ARTHRITIS & RHEUMATISM, JOHN WILEY & SONS, INC, US, vol. 52, no. 4, 1 April 2005 (2005-04-01), pages 1314-1318, XP002596196, ISSN: 0004-3591, DOI: 10.1002/ART.20975 [retrieved on 2005-04-07]
- SPAGNOU S ET AL: "Lipidic carriers of siRNA: differences in the formulation, cellular uptake, and delivery with plasmid DNA", BIOCHEMISTRY, AMERICAN CHEMICAL SOCIETY, US, vol. 43, no. 42, 28 September 2004 (2004-09-28), pages 13348-13356, XP002375949, ISSN: 0006-2960, DOI: 10.1021/BI048950A
- KELLER MICHAEL ET AL: "Intracellular delivery of nucleic acids: Differences between transfection and siFection reflect differences between DNA and RNA, and between oligodeoxynucleotides and oligonucleotides", NON-VIRAL GENE THERAPY: GENE DESIGN AND DELIVERY SPRINGER-VERLAG TOKYO, 37-3, HONGO 3-CHOME BONKYO-KU, TOKYO, 113, JAPAN, 2005, pages 441-455,
- AMY C RICHARDS GRAYSON ET AL: "Biophysical and Structural Characterization of Polyethylenimine-Mediated siRNA Delivery in Vitro", PHARMACEUTICAL RESEARCH, KLUWER ACADEMIC PUBLISHERS-PLENUM PUBLISHERS, NL, vol. 23, no. 8, 15 July 2006 (2006-07-15), pages 1868-1876, XP019405163, ISSN: 1573-904X, DOI: 10.1007/S11095-006-9009-2

### SEQUENCE LISTING

<110> Centre National de la Recherche Scientifique (CNRS) Institut National de la Santé et de la Recherche Médicale (INSERM)
<120> Compositions comprising a siRNA and lipidic 4,5-disubstituted 2-deoxystreptamine ring aminoglycoside derivatives and uses thereof
<130> D24806
<140> PCT/EP2007/060571
   <141> 2007-10-04
<150> US 60/849,000 <151> 2006-10-04
<160> 4
<170> PatentIn version 3.3
<210> 1
   <211> 22
   <212> RNA
   <213> Artificial sequence
<220>
   <223> anti-GFP siRNA
<400> 1
   gcaagcugac ccugaaguuc au 22
<210> 2
   <211> 16
   <212> DNA
   <213> Artificial sequence
<220>
   <223> GFP reverse PCR primer
<400> 2
   cgggcatggc ggactt 16
<210> 3
   <211> 15
   <212> DNA
   <213> Artificial sequence
<220>
   <223> GFP PCR probe
<400> 3
   cagcacgact tcttc 15
<210> 4
   <211> 20
   <212> DNA
   <213> Artificial sequence
<220>
   <223> GFP forward PCR primer
<400> 4
   gctaccccga ccacatgaag 20

## Description

### FIELD OF THE INVENTION

The present invention relates to a composition comprising a siRNA and a transfecting compound consisting of an aminoglycoside of the class of 4,5-disubstituted 2-deoxystreptamine ring linked via a spacer molecule to a lipid moiety of formula -(R1)R2 wherein R1 and R2 represent, independently of one another, a hydrogen atom or a fatty aliphatic chain or R1 or R2 is absent, with the proviso that at least one of R1 and R2 represents a fatty aliphatic chain; or -OR3 or -NR3 wherein R3 represents a steroidal derivative. The invention also concerns in vitro and in vivo applications of these compositions.

### BACKGROUND ART

RNA interference (RNAi) has become a powerful and widely used tool for the knockdown of target gene expression via a posttranscriptional silencing process and subsequent phenotypic analysis of gene function in cells. Therapeutic approaches involving RNA interference mechanism are also actively under investigation. To achieve efficient target gene knockdown, specific 21-25 double-stranded small interfering RNA (siRNA) molecules have to be delivered into the cytoplasmic compartment of the cell.

Different methods have been used to deliver siRNA into cells. Among them, the most widely used is based on the use of common cationic lipids which were previously developed for DNA transfection in the 90's. These cationic lipids are composed of a hydrophobic moiety linked to a cationic headgroups including a quaternary ammonium such as dioctadecyldiammonium bromide (DODAB), or dioleoyl trimethylammonium propane (DOTAP), or a polycation such as dioctadecylamidoglycylspermine (DOGS), or guanidinium residues such as bis(guanidinium)-tren-cholesterol (BGTC). For instance, WO2005/014837 (8) discloses the use of lipopolyamine cationic lipids (such as compound DOGS) for the transfection of siRNAs.
Other types of cationic lipids were also synthesized for DNA transfection. Notably, compounds of formula A-Y-L, wherein A is an aminoglycoside, Y a spacer molecule and L a lipid moiety have been described in US patent application N° 10/228,959 published under number US 2003-0054556 A1 (1). The interest of these compounds for DNA transfection has further been confirmed in Sainlos et al, 2005 (2), Sainlos et al, 2004 (3) and Belmont et al, 2002 (4).

A co-lipid like dioleoyl phosphatidyl ethanolamine (DOPE) is usually combined with the cationic lipids to form liposomes. The electrostatic interactions between the plasmid DNA and cationic headgroups lead to the formation of particles whose structure has been shown to be lamellar or hexagonal depending on the co-lipid used and its proportion in the cationic liposomes.

Nevertheless, the structural characteristics of complexes formed with siRNA are still unknown, and the relevance of the structure observed with plasmid DNA remains to be elucidated for siRNA. It should also be stressed that all cationic vectors developed for DNA transfection are not necessary optimized for the delivery of nucleic acids of low molecular weight and specific chemical structure such as siRNA molecules.

Indeed, siRNA molecules display a very specific structure, very different from that of DNA generally used in DNA transfection. A siRNA is a short (usually 21-25 nucleotides) double-strand of RNA (dsRNA) with a few nucleotides (usually 2) 3' overhangs on either end. Each strand has a 5' phosphate group and a 3' hydroxyl (-OH) group. This very specific structure of naturally occuring siRNAs is the result of processing by Dicer, an enzyme that converts either long dsRNAs or hairpin RNAs into siRNAs. This particular structure has been conserved for synthetic siRNAs. In contrast, transfected DNA are usually used for expressing a particular gene, which implies that the transfected DNA molecule comprises a sequence encoding said gene, as well as regulatory sequences for directing the expression of the gene into the transfected cell. Tranfected DNA molecules are thus generally long length DNA molecules, contrary to very short 21-25 nucleotides siRNAs. This very significant structure difference may result in a significant change in the transfecting efficiency of a particular tranfecting compound with one nucleic acid category or the other.

In addition, the activities of DNA expression vectors and siRNA, once transfected into a target cell, are completely different and mediated by distinct mechanisms. DNA expression vectors have to be transcribed to results in gene expression, while siRNAs assemble into endoribonuclease-containing complexes known as RNA-induced silencing complexes (RISCs), unwinding in the process, and siRNA strands subsequently guide the RISCs to complementary RNA molecules, where they cleave and destroy the cognate RNA (effecter step), thus inhibiting a particular gene expression. Cleavage of cognate RNA takes place near the middle of the region bound by the siRNA strand. The action mechanism of siRNAs is thus quite complex, and efficient transfecting compounds for siRNAs should thus allow for both the entry of siRNAs into the target cells and the release of functional siRNAs in the cell to permit an efficient interference.

Due to the particularities of siRNAs in term of structure and action mechanism, it is clear that cationic lipids which have been optimized for DNA transfection, although some of them may have some efficiency in tranfecting siRNA also, may not all be adapted to siRNAs transfection. Nevertheless, most currently known transfecting compounds have been optimized for DNA transfection there has not been many studies yet intending to optimized transfecting compounds for siRNAs transfection. There is thus a need for transfecting compounds specialized for tranfecting siRNA with a high efficiency.

The inventors have explored the efficiency of lipidic aminoglycoside derivatives to deliver siRNA molecules and knockdown gene expression in mammalian cells. They successfully synthesized new cationic lipids based upon the use of aminoglycoside as cationic headgroup linked to dioleoyl chains via a succinyl spacer. Aminoglycosides consist of oligosaccharides decorated with up to six amino groups as well as numerous hydroxyl groups thus providing a versatile polycationic frame work. The inherent flexibility of the glycoside bonds of the aminoglycosides can remodel themselves to interact with RNA molecules. This family offers the advantageous possibility to modify the cationic headgroup using different aminoglycosides as kanamycine, tobramycine, neomycine or paromomycine, known to interact with the major groove of duplex RNA or more generally with A-form nucleic acids.

The inventors found that while complexation of siRNA molecules with lipidic aminoglycosides derivatives adhere to the three stage model of colloidal stability previously proposed for plasmid DNA, siRNA complexes exhibited a smaller mean diameter and a total siRNA entrapment for lower lipidic aminoglycoside derivatives/siRNA charge ratio than that observed with siRNA complexed with guanidinium-cholesterol reagent.

They also found that lipidic aminoglycoside derivatives/siRNA complexes form specific structures consisting of self-assembled RNA-condensed particles with various morphologies depending on the aminoglycosides used as polar headgroup. Indeed, while lipidic aminoglycoside derivatives of the class of 4,6-disubstituted 2-deoxystreptamine ring formed usual "onion-like" structures made of a regular superimposition of lipid bilayer and siRNA molecules, lipidic aminoglycoside derivatives of the class of 4,5-disubstituted 2-deoxystreptamine ring surprisingly formed distinct structures with grape of small condensed structures.

In addition, comparative analysis of *in vitro* gene expression inhibition with lipidic aminoglycoside derivatives and cationic lipids designed for DNA transfection indicated that lipidic aminoglycoside derivatives of the class of 4,5-disubstituted 2-deoxystreptamine ring had the best efficiency to induce the interference mechanism into target cells. Indeed, although all lipidic aminoglycoside derivatives and DNA transfection reagent BGTC all allowed for a high siRNA internalization, only aminoglycoside derivatives of the class of 4,5-disubstituted 2-deoxystreptamine ring surprisingly permitted a high siRNA mediated RNAi knockdown of the target gene. Although the reasons of this phenomenon are still unknown, it may be postulated that the distinct structures of this class of that lipidic aminoglycoside derivatives/siRNA complexes may be involved in the significantly higher efficiency of these particular compounds.

Finally, comparative analysis of *in vitro* gene expression inhibition with lipidic aminoglycoside derivatives and commercially available reagents for siRNA delivery also demonstrated that lipidic aminoglycoside derivatives of the class of 4,5-disubstituted 2-deoxystreptamine ring displayed a higher efficiency for siRNA mediated RNAi knockdown than currently commercially available reagents for siRNA delivery.

Thus, among the general class of lipidic aminoglycoside derivatives known to mediate efficient DNA transfection, the inventors have identified a new subclass of 4,5-disubstituted 2-deoxystreptamine ring lipidic aminoglycoside derivatives particularly well adapted to the efficient and functional delivery of siRNA into mammalian cells.

### DESCRIPTION OF THE INVENTION

The invention thus concerns a composition comprising a siRNA and a transfecting compound of general formula (I):

A-Y-L (I),

wherein
- A represents an aminoglycoside of the class of 4,5-disubstituted 2-deoxystreptamine ring,
- Y represents a spacer, and
- L represents:
   - either a radical -(R1)R2, wherein R1 and R2 represent, independently of one another, a hydrogen atom or a fatty aliphatic chain or R1 or R2 is absent, with the proviso that at least one of R1 and R2 represents a fatty aliphatic chain,
   - or a radical -N-R3 or -O-R3, wherein R3 represents a steroidal derivative
or a salt, an isomer or a mixture thereof.

For the purpose of the present invention, the term "aminoglycoside" (or "aminosides") is intended to mean natural heterosides formed by the combining of a genin of the aminocyclitol group with generally several saccharides, at least one of which is an amino sugar (osamine). Aminoglycosides may differ both in the molecular nucleus, which can be streptamine or 2-deoxystreptamine, and in the aminohexoses linked to the nucleus. Most currently used aminoglycosides have 2-deoxystreptamine as molecular nucleus and can be further segregated into two subclasses, depending on the positions of the 2-deoxystreptamine ring II to which are attached the other rings:
- 4,5-disubstituted 2-deoxystreptamine aminoglycosides in which the 2-deoxystreptamine nucleus is substituted in positions 4 and 5, and
- 4,6-disubstituted 2-deoxystreptamine aminoglycosides in which the 2-deoxystreptamine nucleus is substituted in positions 4 and 6.

The 4,5-disubstituted class includes neomycin, paromomycin, and ribostamycin, while the 4,6-disubstituted class includes tobramycin, kanamycine, amikacin and gentamicin.

The tranfecting compound of the composition according to the invention is a 4,5-disubstituted 2-deoxystreptamine aminoglycoside, as defined above. It may advantageously be selected from paromomycin, neomycin, and ribostamycin, preferably from paromomycin and neomycin. Alternatively, any synthetic or semisynthetic 4,5-disubstituted 2-deoxystreptamine aminoglycoside, as defined above, may be used instead.

According to the present invention, the term "spacer" is intended to mean any chemical group which makes it possible both to form the bond between the aminoglycoside or its polyguanidylated derivative and the lipid component of the molecule, and to distance these two components in order to reduce any undesired interaction between them. Consequently, the spacer is a difunctional chemical group which may, for example, consist of one or more chemical functions chosen from alkyls having 1 to 6 carbon atoms, ketone, ester, ether, amine, amide, amidine, carbamate or thiocarbamate functions, glycerol, urea, thiourea or aromatic rings.

For instance, the spacer may be selected from the radicals of formula:

-C(O)-;

-NH-C(O)-CH₂-CH₂-;

-W-(CH₂-)ₖ-W'-;

and

-(CH₂-)ᵢ-W-(CH₂)ⱼ-

in which i, j and k are integers chosen between 1 and 6 inclusive, and W and W' are groups, which may be identical or different, chosen from ketone functions, ester functions, ether functions, amino functions, amide functions, amidine functions, carbamate functions, thiocarbamate functions, glycerol, urea, thiourea, and aromatic rings.

For the purpose of the present invention, the term "fatty aliphatic chains" is intended to mean saturated or unsaturated alkyl radicals containing 10 to 22 carbon atoms and optionally containing one or more hetero atoms, provided that said fatty aliphatic chains have lipid properties. Preferably, they are linear or branched alkyl radicals containing 10 to 22 carbon atoms and 1, 2 or 3 unsaturations. Preferably, said alkyl radicals comprise 10, 12, 14, 16, 18, 20 or 22 carbon atoms, more preferably 18, 20 or 22 carbon atoms. Mention may be made more particularly of aliphatic radicals selected from aliphatic radicals -(CH₂)₁₁CH₃, -(CH₂)₁₃CH₃,-(CH₂)₁₅CH₃, -(CH₂)₁₇CH₃, myristoyl, oleyl, and stearyl.

In a preferred embodiment, when L represents -(R1)R2, L is selected from dimyristoyl, dioleyl, and distearyl.

For the purpose of the present invention, the term "steroidal derivatives" is intended to mean polycyclic compounds of the cholestane type. These compounds may or may not be natural and are preferably selected from cholesterol, cholestanol, 3-α-cyclo-5-α-cholestan-6-β-ol, cholic acid, cholesteryl formiate, cholestanyl formiate, 3-α-5-cyclo-5-α-cholestan-6-β-yl formiate, cholesterylamine, 6-(1,5-dimethylhexyl)-3a,5a-dimethylhexadecahydrocyclopenta[a]cyclopropa[2,3]cyclopenta[1,2-f]naphthalen-10-ylamine, and cholestanylamine.

Preferred transfecting compounds used in compositions according to the invention are :
dioleylamine-A-succinyl-neomycine ("DOSN") of formula: 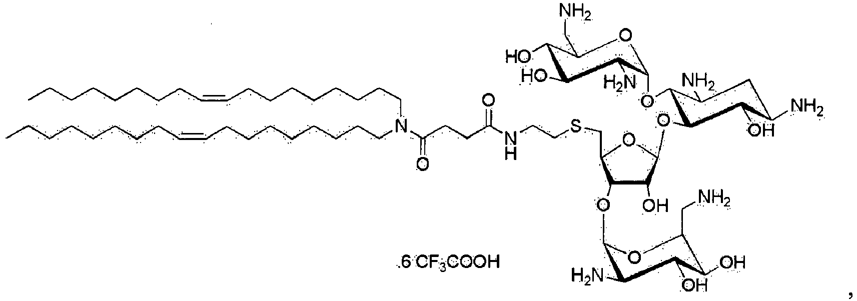
dioleylamine-A-succinyl-paromomycine ("DOSP") of formula: 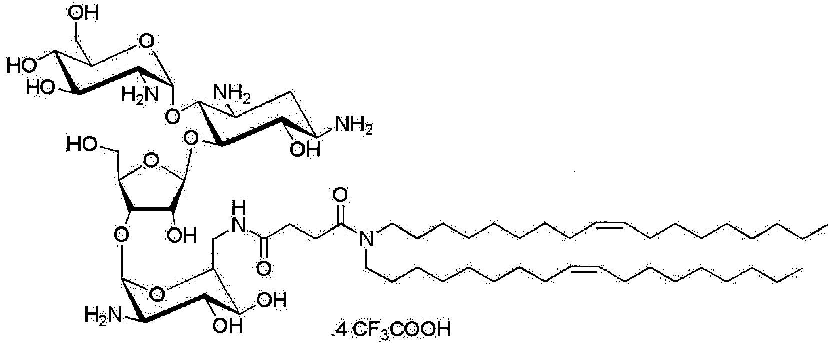
NeoChol of formula: 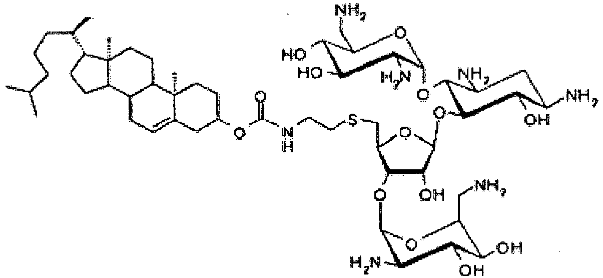
NeoSucChol of formula: 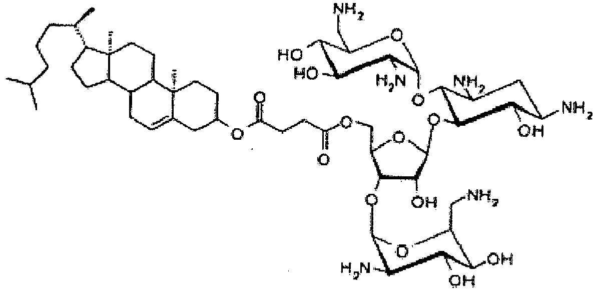
ParomoChol of formula: 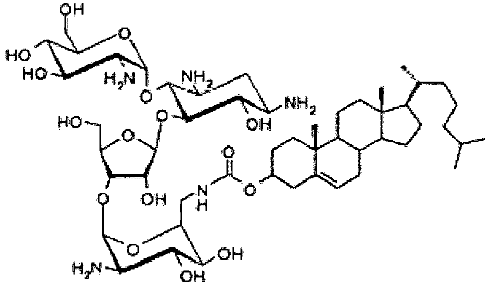
ParomoCapSucDOLA of formula: 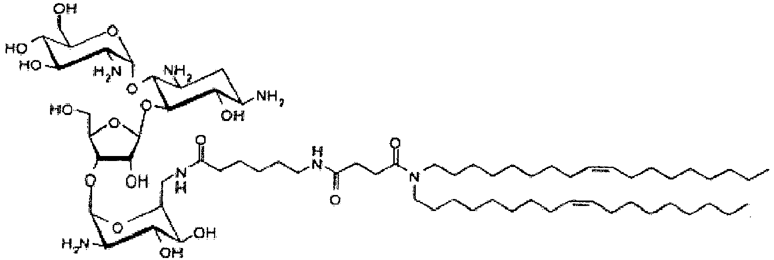
ParomoLysSucDOLA of formula: 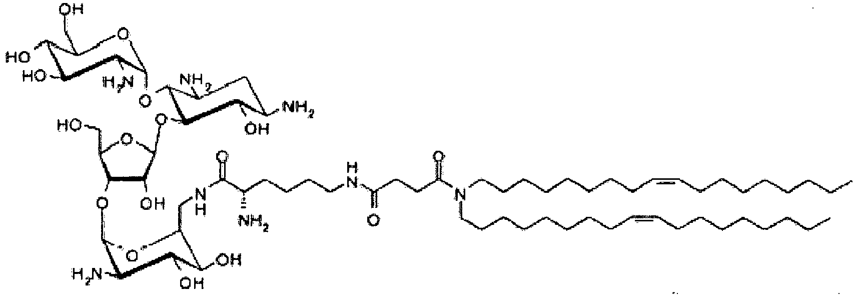
NeodiSucDODA of formula: 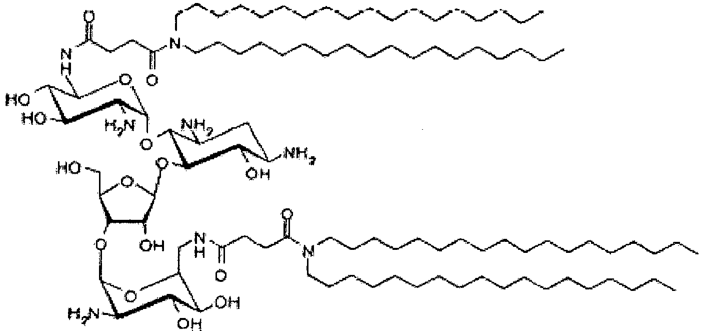
NeodiLysSucDOLA of formula: 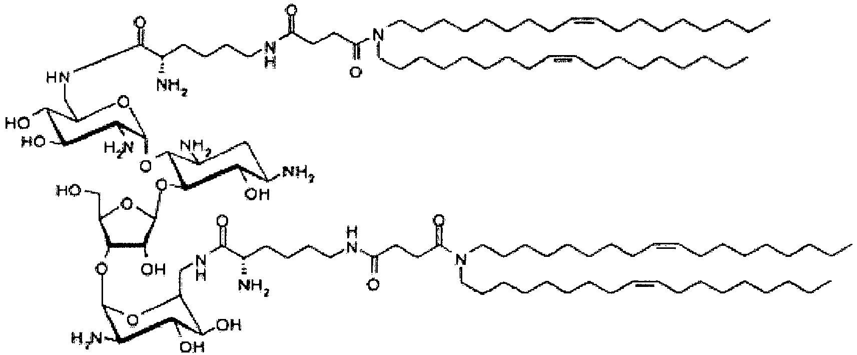
and [ParomoLys]₂-Glu-Lys-[SucDOLA]₂ of formula:

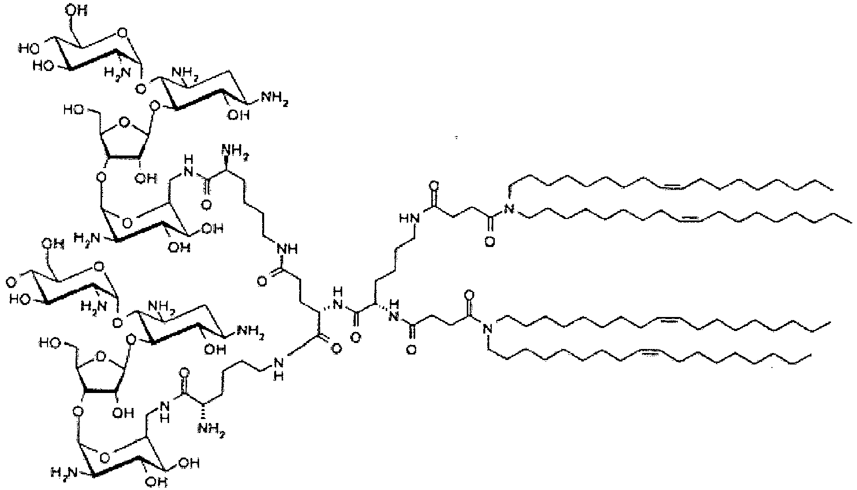

It is to be understood that the present invention also relates to compositions comprising the isomers of the transfecting compounds of general formula (I) when they exist, and also the mixtures thereof, or the salts thereof.

In particular, the compounds of the invention may be in the form of nontoxic and pharmaceutically acceptable salts. These nontoxic salts comprise salts with inorganic acids (for example hydrochioric, sulfuric, hydrobromic, phosphoric, nitric acid) or with organic acids (acetic, propionic, succinic, maleic, hydroxymaleic, benzoic, fumaric, methanesulfonic, trifluoroacetic or oxalic acid).

The compounds of general formula (I) according to the present invention are prepared as previously described in US 2003-0054556 A1 and in Sainlos et al, 2005 (2), Sainlos et al, 2004 (3) and Belmont et al, 2002 (4).

The compositions according to the invention may also comprise one or more adjuvants capable of associating with the transfecting compound/siRNA complexes and improving the transfecting power thereof. In another embodiment, the present invention therefore relates to compositions comprising a siRNA, a transfecting compound as described above and at least one adjuvant capable of associating with the transfecting compound/ siRNA complexes and improving the transfecting power thereof.

These adjuvants which make it possible to increase the transfecting power of the compounds according to the present invention are advantageously chosen from lipids (for example neutral lipids, in particular phospholipids), peptides (for example histone, nucleolin or protamine derivatives such as those described in WO 96/25508) , proteins (for example proteins of the HNG type such as those described in WO 97/12051) and/or polymers (for example polymers which make it possible to turn the transfecting compound/ siRNA formulations into "stealth" formulations, such as polyethylene glycol (PEG) introduced into, the formulation on its own or in a form attached to a lipid in order to colloidally stabilize the transfecting compound/ siRNA formulations, for example PEG-cholesterol).

From this point of view, the compositions of the invention may comprise, as an adjuvant, one or more neutral lipids which in particular have the property of forming lipid aggregates. The term "lipid aggregate" is a generic term which includes liposomes of all types (both unilamellar and multilamellar) and also micelles or more amorphous, aggregates.

More preferentially, the neutral lipids used in the context of the present invention are lipids containing two fatty chains. Particularly advantageously, use is made of natural or synthetic lipids which are zwitterionic or lacking ionic charge under physiological conditions. They may be chosen more particularly from dio leoylphosphatidylethano lamine (DOPE), oleoylpalmitoylphosphatidylethanolamine (POPE), distearoyl-, dipalmitoyl- and dimirystoylphosphat±dylethanolamines and also the derivatives thereof which are N-methylated 1 to 3 times, phosphatidylglycerols, diacylglycerols, glycosyldiacylglycerols, cerebrosides (such as in particular galactocerebrosides), sphingolipids (such as in particular sphingomyelins) or asialogangliosides (such as in particular asialoGM1 and asialoGM2). According to a particularly preferred alternative, the lipid adjuvants used in the context of the present invention are chosen from DOPE, DOPO, cholesterol or the lipid derivatives of nonionic surfactants, such as PEG-cholesterol.

These various lipids may be obtained either by synthesis or by extraction from organs (for example the brain) or from eggs, using conventional techniques well known to those skilled in the art. In particular, the extraction of natural lipids may be carried out using organic solvents.

Preferentially, the compositions of the invention comprise from 0.01 to 20 equivalents of adjuvant per equivalent of siRNA in mol/mol, and more preferentially from 0.5 to 5 molar equivalents , corresponding to a charge ratio (moles of positive charges)/(moles of negative charges) comprised between 0.01 and 20, preferentially between 0.5 and 5.

According to another alternative, the adjuvants cited above make it possible to improve the transfecting power of the compositions according to the present invention; in particular, the peptides, the proteins or certain polymers, such as polyethylene glycol, may be conjugated to the transfecting compounds according to the invention, and not simply mixed. In particular, the PEG may be attached covalently to the lipid derivatives of aminoglycoside according to the present invention either at a remaining amine of the aminoglycoside or at the hydroxyl radicals of the aminoglycoside.

According to a particularly advantageous embodiment, the compositions according to the present invention also comprise a targeting element which makes it possible to direct the transfer of the siRNA. This targeting element may be an extracellular targeting element which makes it possible to direct the transfer of the siRNA toward certain desired cell types or tissues (tumor cells, hepatic cells, hematopoietic cells, etc.). It may also be an intracellular targeting element which makes it possible to direct the transfer of the siRNA toward certain preferred cellular compartments (mitochondria, nucleus, etc.). The targeting element may be mixed with the transfecting compounds according to the invention and with the siRNAs and, in this case, the targeting element is preferably attached covalently to a fatty aliphatic chain (at least 10 carbon atoms) or to a polyethylene glycol. According to another alternative, the targeting element is covalently attached to the transfecting compound according to the invention, either on one of the chemical functions which make up the spacer Y, or at the end of the lipid component (for example at the end of R and/or R when they represent fatty aliphatic chains), or directly on the aminoglycoside at one of the remaining amines or at the hydroxyl radicals. Finally, the targeting element may also be attached to the siRNA, as specified previously.

Among, the targeting elements which can be used in the context of the invention, mention may be made of sugars, peptides, proteins, oligonucleotides, lipids, neuromediators, hormones, vitamins or derivatives thereof. Preferentially, they are sugars, peptides, vitamins or proteins such as, for example, antibodies or antibody fragments, cell receptor ligands or fragments thereof, receptors or receptor fragments.

For example, they may be ligands for growth factor receptors, for cytokine receptors, for receptors of the cellular lectin type or for folate receptors, or ligands containing an RGD sequence with an affinity for adhesion protein receptors such as integrins. Mention may also be made of transferrin receptors, HDL receptors and LDL receptors or the folate transporter. The targeting element may also be a sugar which makes it possible to target lectins such as the receptors for asialoglycoproteins or for sialidized species, such as sialyl Lewis X, or an Fab antibody fragment or a single-chain antibody (ScFv).

The present description also discloses the use of the compositions according to the present invention, for transferring nucleic acids into cells in vitro, in vivo or ex vivo.

More precisely, an object of the present invention is the use of compositions according to the invention, for preparing a medicinal product intended to treat diseases, in particular diseases which result from the overrexpression of a gene product. The siRNA contained in said medicinal product specifically targets the mRNA of said gene product, thereby correcting said diseases in vivo or ex vivo.

The present description also discloses a method for inhibiting a target gene expression in a subject, comprising administering to said subject a composition according to the invention, wherein the siRNA comprised in said composition is specifically directed to said target gene.

For uses in vivo, for example in therapy or for studying gene regulation or creating animal models of pathological conditions, the compositions according to the invention may be formulated with a view to topical, cutaneous, oral, rectal, vaginal, parenteral, intranasal, intravenous, intramuscular, subcutaneous, intraocular, transdermal, intratracheal, intraperitoneal, etc. administration.

Preferably, the compositions of the invention contain a vehicle which is pharmaceutically acceptable for an injectable formulation, in particular for direct injection into the desired organ, or for topical administration (on skin and/or mucous membrane).

They may in particular be sterile, isotonic solutions or dry compositions, in particular lyophilized compositions, which, by adding, as appropriate, sterilized water or physiological saline, allow injectable solutes to be made up. The doses of siRNAs used for the injection and also the number of administrations may be adjusted depending on various parameters, and in particular depending on the method of administration used, on the pathological condition in question, on the gene to be expressed or on the desired duration of the treatment. With regard more particular to the method of administration, it may be either a direct injection into the tissues, for example into the tumors, or an injection into the circulatory system, or it may involve treating cells in culture followed by their reimplantation in vivo, by injection or transplantation. The relevant tissues in the context of the present invention are, for example, muscles, skin, brain, lungs, trachea, liver, spleen, bone marrow, thymus, heart, lymph, blood, bones, cartilage, pancreas, kidneys, bladder, stomach, intestines, testicles, ovaries, rectum, nervous system, eyes, glands, connective tissues, etc.

Concerning in vitro applications, the invention further relates to a method for transferring a siRNA into cells in vitro, comprising:
(1) providing a composition according to claim 1, and
(2) bringing the cells into contact with said composition.

### DESCRIPTION OF THE FIGURES

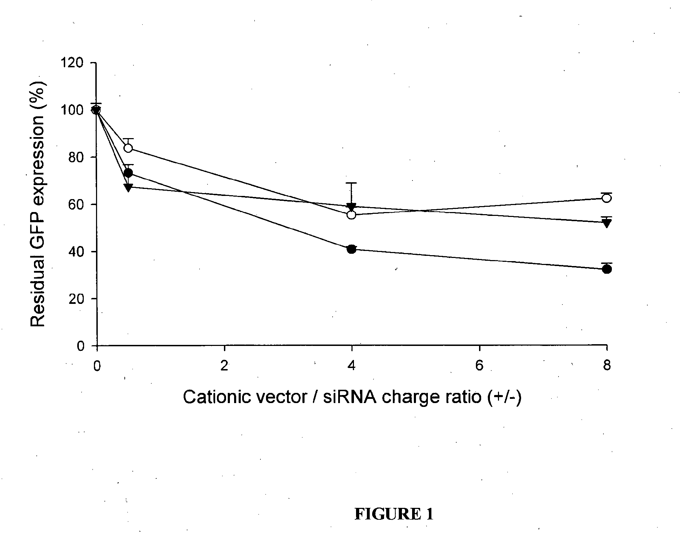

**Figure 1** : *In vitro* GFP inhibition of d2-GFP cells by RNA interference. Transfections were performed with siRNA targeted to GFP complexed with BGTC-DOPE (●), DCChol-DOPE (▼) and PEI (○) at various charge ratios +/- ranging from 0 to 8. *In vitro* transfection was performed in 500 µl of serum-free medium for two hours with 0,39 µg siRNA/well. Then 500 µl of medium containing 20% FCS was added in each well. GFP fluorescence measurement was carried out 24 hours post-transfection. Each point represents the mean value and SEM of six individual values.

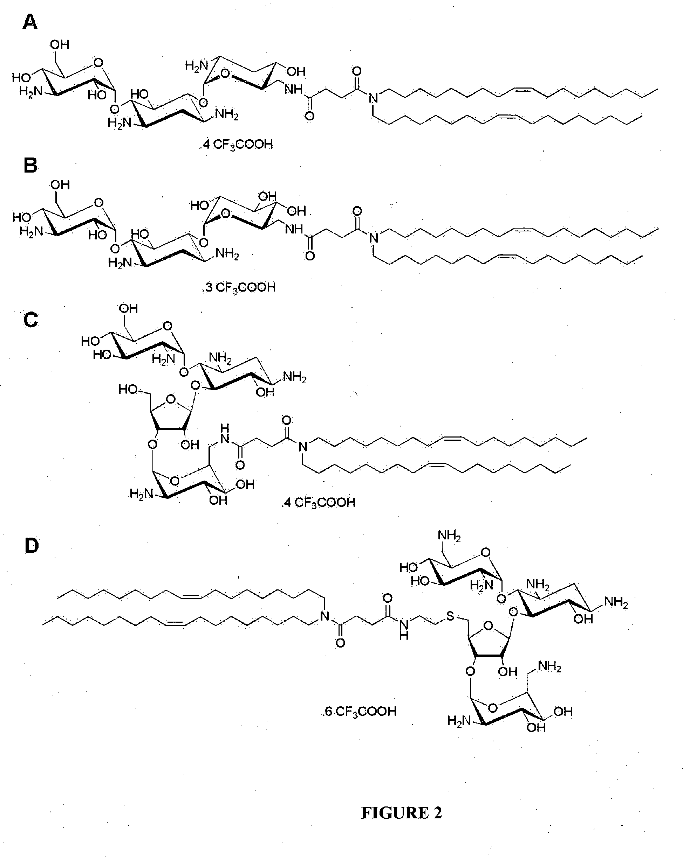

**Figure 2** : Structure of lipidic aminoglycoside derivatives. (**A**) DiOleylamine A-Succinyl-Tobramycine (DOST), (**B**) DiOleylamine A-Succinyl-Kanamycine (DOSK), (**C**) DiOleylamine A-Succinyl-Paromomycine (DOSP) and (**D**) DiOleylamine A-Succinyl-Neomycine (DOSN).

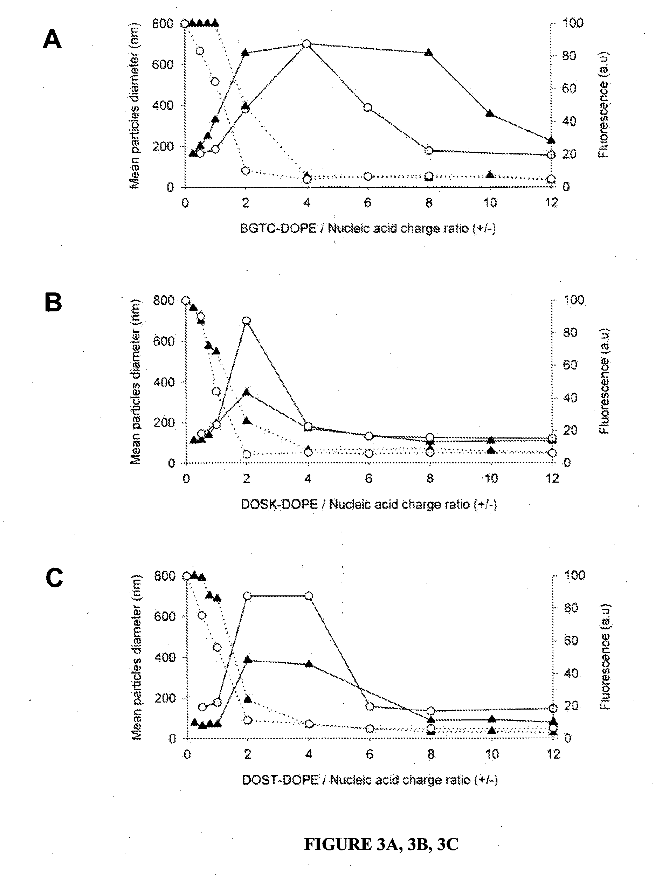

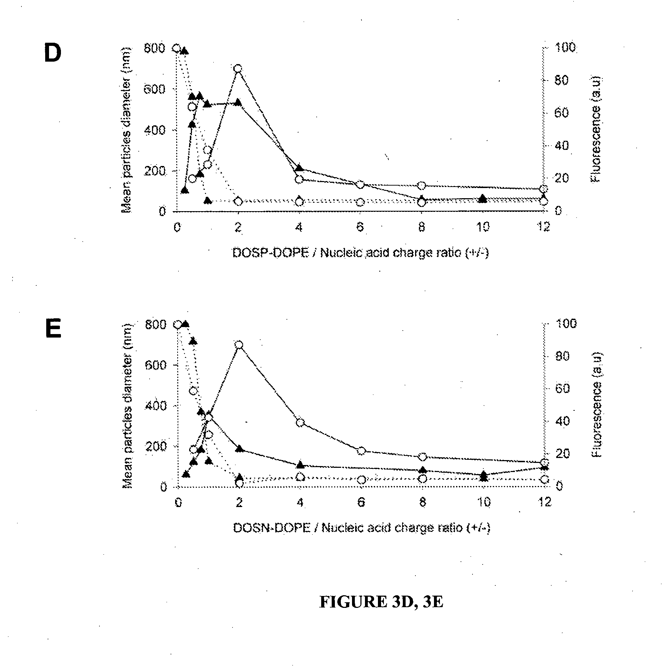

**Figure 3** : Physicochemical properties of complexes resulting from the association of various cationic lipids with plasmid DNA or siRNA. Nucleic acids were complexed with BGTC-DOPE (**A**), DOST-DOPE (**B**), DOSK-DOPE (**C**), DOSP-DOPE (**D**), DOSN-DOPE (**E**) by mixing cationic liposomes at different concentrations with plasmid DNA (○) or siRNA (A). To assess the colloidal stability of complexes and nucleic acids complexation, dynamic light scaterring (solid line) and BET fluorescence measurements (dashed line) were performed after 1 hour of complexation, respectively. Arbitrary value of 700 nm was attributed to complexes that were not colloidally stable.

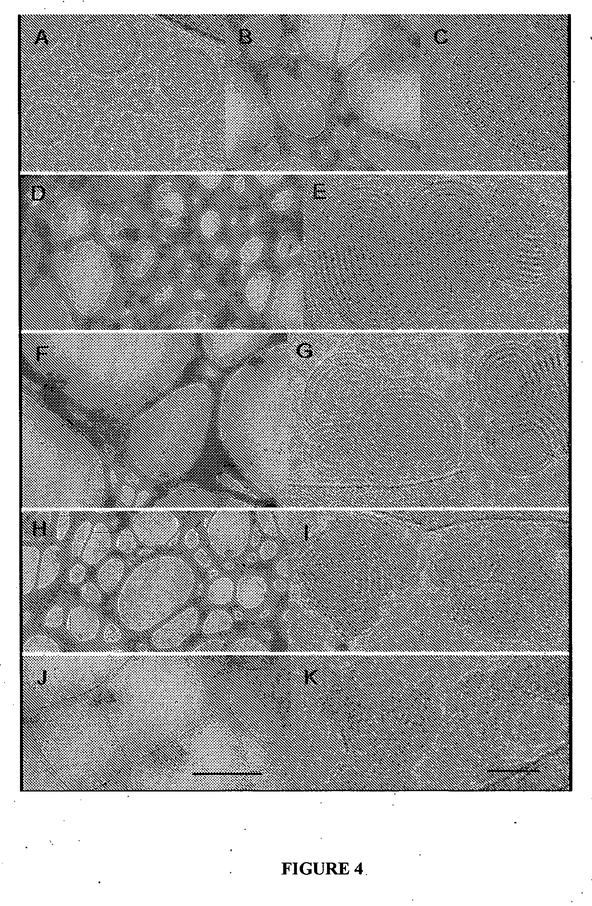

**Figure 4** : Gallery of cationic lipid/siRNA complexes observed by cryo-electron microscopy. (A) Unilamellar BGTC-DOPE liposomes. (B) Field of view of BGTC-DOPE/siRNA complexes. C) Structure of a BGTC-DOPE/siRNA complexes at high magnification. (D) Field of view of DOST-DOPE/siRNA complexes. (E) Structure of a DOST-DOPE/siRNA complexes at high magnification. Note the regular organisation of RNA molecules on the edge. (F) Field of view of DOSK-DOPE/siRNA complexes. (G) Structures of two DOSK-DOPE/siRNA complexes at high magnification. H) Field of view of DOSP-DOPE/siRNA complexes. (I) Structure of two DOSP-DOPE/siRNA complexes at high magnification. (J) Field of view of DOSN-DOPE/siRNA complexes. (K) Structure of two DOSN-DOPE/siRNA complexes at high magnification. Scale bar: 50 nm and 1 µm for high and low magnification images respectively.

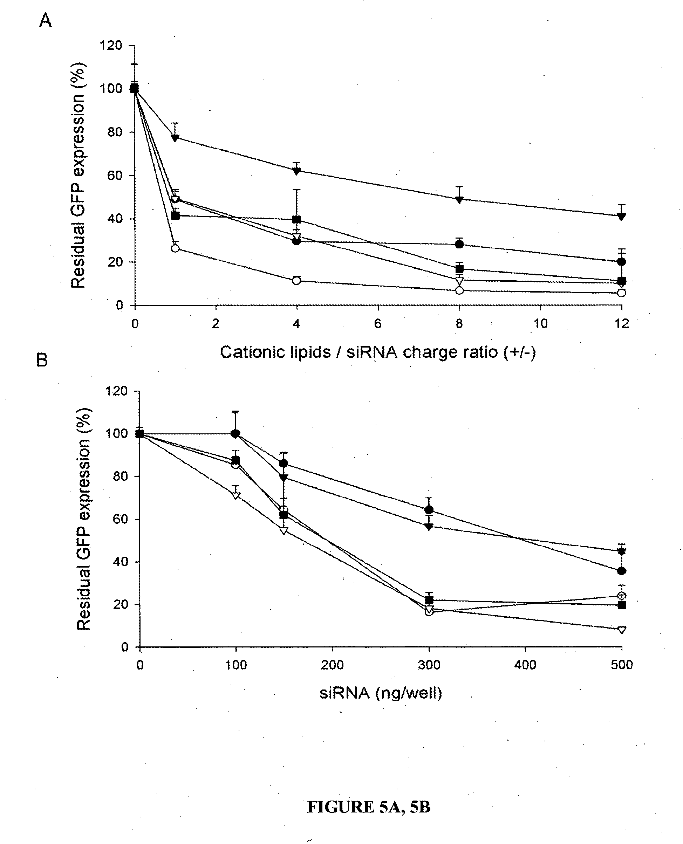

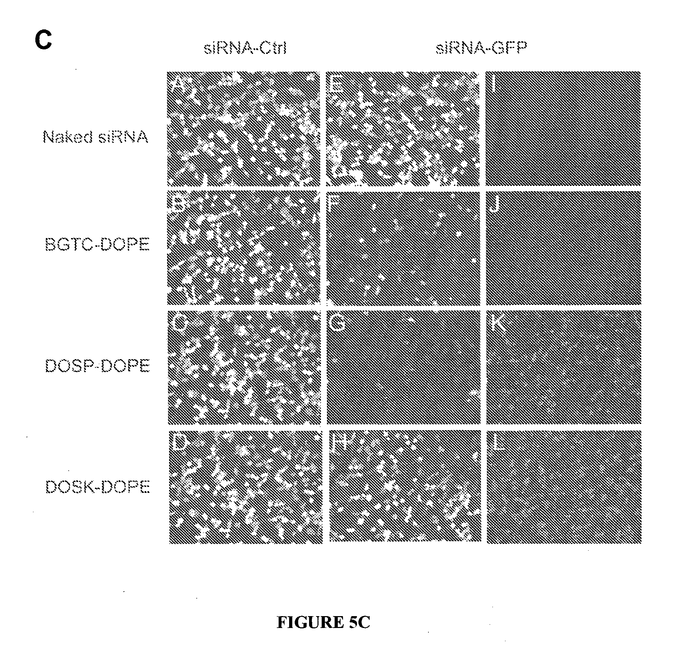

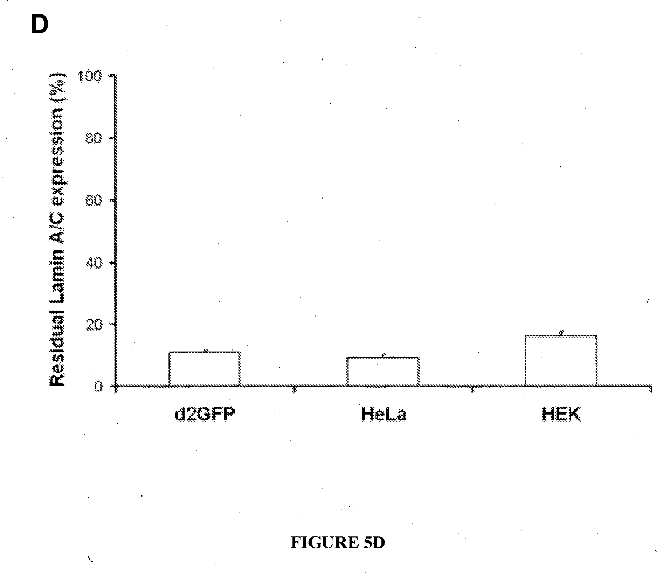

**Figure 5** : GFP expression inhibition by siRNA delivery in d2GFP cells. In vitro transfection efficiency of cationic liposome/siRNA complexes as a function of the charge ratio (**A**) and the amount of siRNA (**B**). In vitro GFP measurement was performed in cells transfected with various cationic liposomes : BGTC-DOPE (●), DOSK-DOPE (▼), DOST-DOPE (o), DOSP-DOPE (■) and DOSN-DOPE (∇). (A) Residual GFP expression expressed as the ratio between GFP fluorescence of transfected cells and cells transfected with non active siRNA as a function of the cationic lipid/siRNA charge ratio (+/-). Cells were transfected with 400 ng siRNA per well (B) Residual GFP expression as a function of the siRNA amount for different vectors. Cationic lipids were used at a charge ratio of 4, 12, 4, 10 and 10 for BGTC-DOPE (●), DOSK-DOPE (▼), DOST-DOPE (o), DOSP-DOPE (■) and DOSN-DOPE (∇), respectively. (**C**) Visualisation of GFP inhibition and siRNA internalisation. Cells were transfected with the Control siRNA (A-D) or the 3'-Rhodamine labelled anti-GFP siRNA (E-L). siRNAs (500 ng/well) were formulated in the absence (Naked siRNA) or in the presence of cationic lipids : BGTC-DOPE, DOSP-DOPE or DOSK-DOPE. Cells were observed under FITC filter to see the GFP fluorescence (A-H) or under Rhodamine filter to see siRNA internalisation. (**D**) Real-time quantitative RT-PCR analysis of human Lamin A/C mRNA after transfection of various human cell lines (HEK, HeLa and d2GFP cells) with DOSP/siRNA lipoplexes, (normalization to HPRT1). Values are relative to cells transfected under the same experimental condition with a control siRNA.

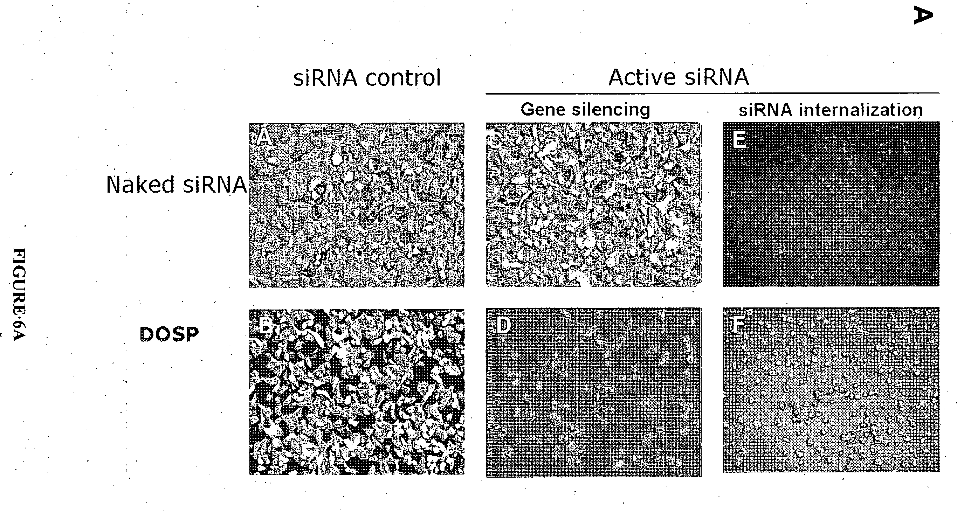

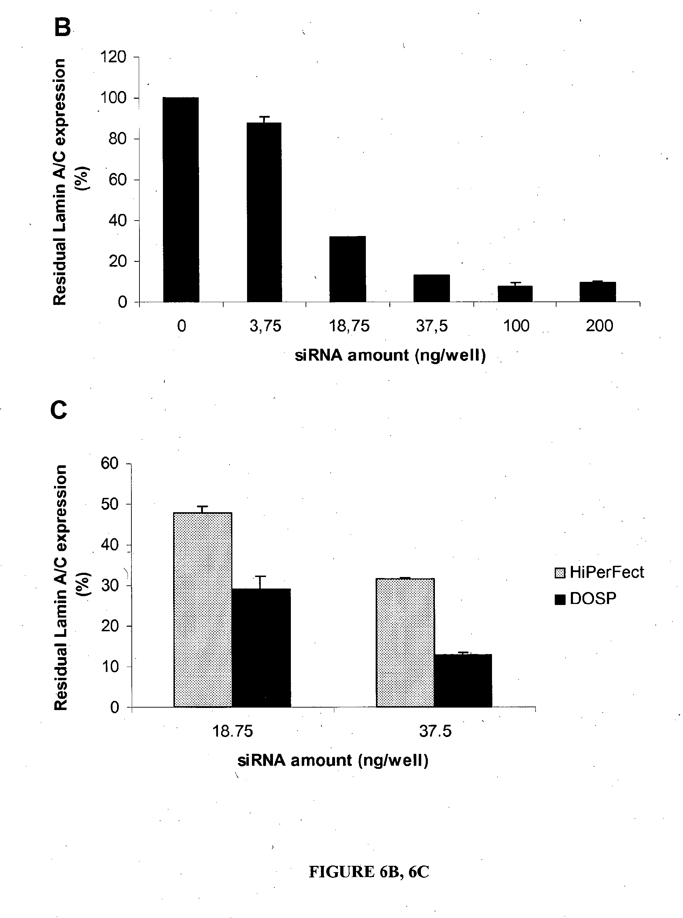

**Figure 6** : Transfection efficiency of aminoglycoside derivative DOSP without a neutral lipid adjuvant.
   **(A) *Fluorescence microscopy visualization of GFP silencing and siRNA internalization.*** The GFP-expressing human lung cancer H1299 cells were transfected with control siRNA (panels *A,B*) or 3'-rhodamine labeled anti-GFP siRNA (panels C-F). The siRNA molecules were formulated in the absence ("naked" siRNA in panels *A,C,E*) or in the presence of DOSP (panels *B,D,F*). The transfected H1299 cells were observed using a FITC filter to visualize GFP fluorescence (panels *A,B,* see white/light grey areas) or a rhodamine filter to visualize siRNA internalization (panels *E,F,* see white/light grey areas).
   **(B) *Lamin A*/*C silencing efficiency using DOSP reagent as a function of siRNA amount.*** Anti-LaminA/C siRNA amounts ranging from 3.75 to 200 ng/well were formulated with DOSP. Results of real-time quantitative RT-PCR analysis of human Lamin A/C mRNA after transfection of HeLa cells showed that DOSP reagent is efficient with very low amount of siRNA (normalization to HPRT1). Values are relative to cells transfected under the same experimental condition with a control siRNA.
   **(C) *Lamin A*/*C silencing efficiency using DOSP reagent or competitor reagent.*** Anti-LaminA/C siRNA at 18.75 and 37.5 ng/well were formulated with DOSP or the commercial reagents HiPerFect. Results of real-time quantitative RT-PCR analysis of human Lamin A/C mRNA after transfection of HeLa cells showed that DOSP reagent was more efficient than HiPerFect (normalization to HPRT1). Values are relative to cells transfected under the same experimental condition with a control siRNA.

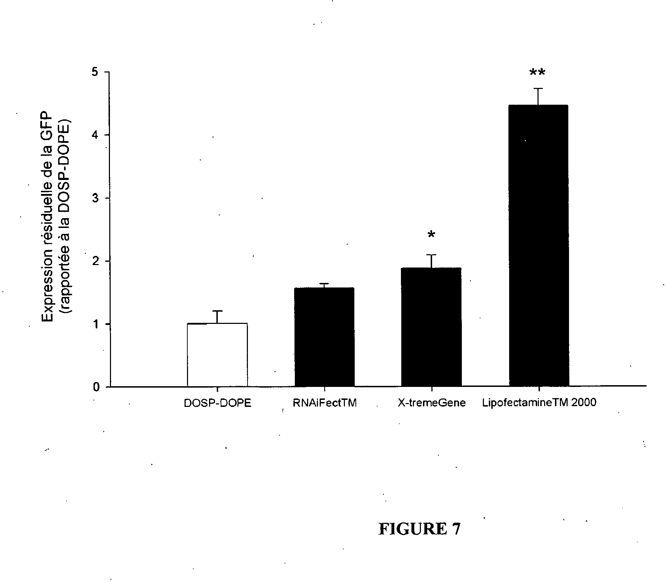

**Figure 7** : Comparison of the efficiency of commercially available reagents for siRNA delivery, with the DOSP-DOPE derivative, for siRNA-mediated GFP gene knockdown in d2GFP cells. 200 ng of anti-GFP siRNA were formulated with appropriate reagent as described by the manufactured and were transfected on 24-well culture plates at cell counts of 65000 cell per well. Asterisks indicate a significant difference (p< ) between cells transfected with the commercially reagent and the DOSP-DOPE.

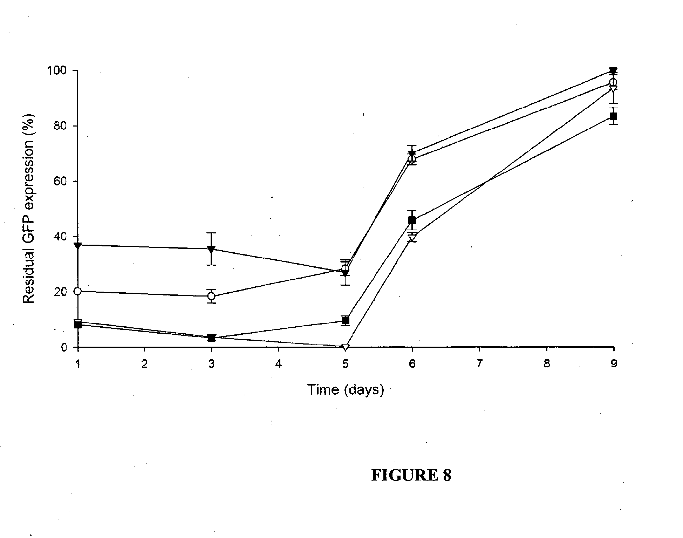

**Figure 8** : Time course of GFP expression after *in vitro* anti-GFP siRNA delivery in d2-GFP. Anti-GFP siRNA was formulated with different vectors : DOSK-DOPE (▼), DOST-DOPE (○), DOSP-DOPE (■) and DOSN-DOPE (∇) ; vectors were used at a +/- charge ratio equal to 4, 12, 4, 10 and 10 respectively. Transfections were performed in 500 µl of serum-free medium for two hours with 0,39 µg siRNA/well. Then 500 µl of medium containing 20% FCS was added in each well. GFP fluorescence measurement was carried at various time. Each point represent the mean value and SEM of six individual values.

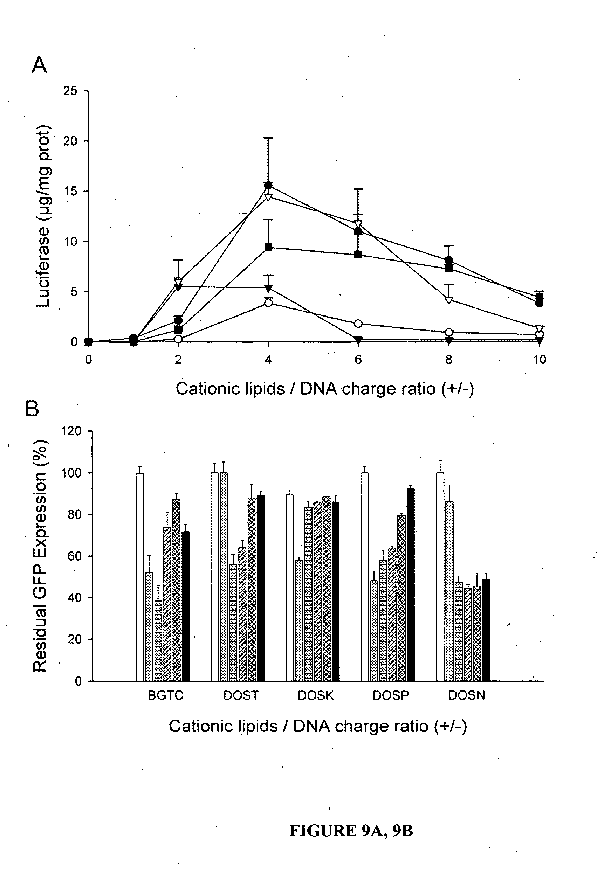

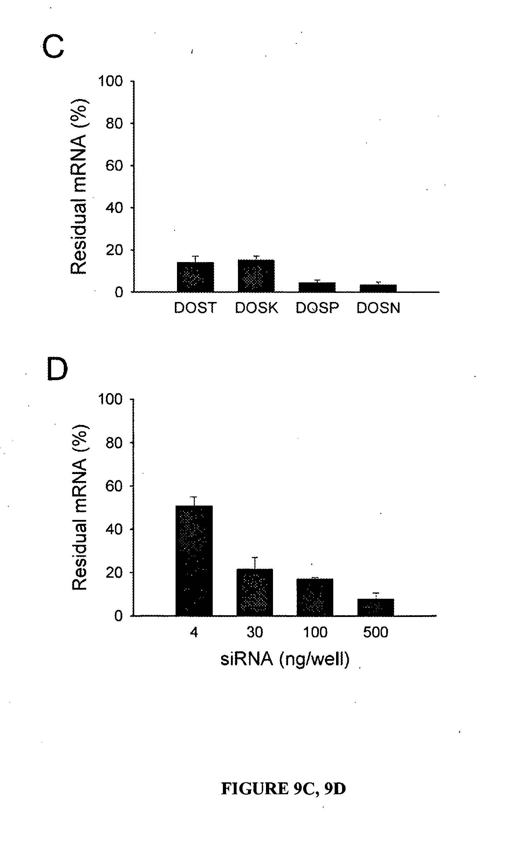

**Figure 9** : *In vitro* plasmid delivery with amino-glycosides derivatives. In vitro transfection were performed in 500 µl of serum-free medium for two hours, replaced then by 1 ml of medium containing 10% FCS. Protein and mRNA measurement was carried out 24 hours post-transfection. Each point represent the mean value and SEM of six individual values. (**A**) Expression of luciferase in d2-GFP cells as a function of the +/- charge ratios. Transfections were performed with 1 µg of luciferase plasmid complexed with cationic lipid : BGTC-DOPE (●), DOSK-DOPE (▼), DOST-DOPE (○), DOSP-DOPE (■) and DOSN-DOPE (∇). (**B**) Expression of the siRNA hairpin encoded by the pTER plasmid. Transfection were performed with 1 µg of pTER plasmid complexed with cationic lipid at various +/charge ratios : 0 (empty bars), 2, (grey bars), 4 (horizontal line), 6 (diagonal line bars), 8 (crossed line bars), 10 (filled bars). (**C and D**) Co-delivery of the KCNE1 plasmid and the siRNA targeted the KCNE1 in COS-7 cells. 0,8 µg of KCNE1 plasmid and 0,8 µg (C) or the desired amount (D) of siRNA targeted KCNE1, were mixed then complexed with the appropriate amino-glycosides derivatives (C) or the DOSP-DOPE cationic lipid only (D). Results were expressed as the percentage of KCNE1 mRNA quantity obtained in the various conditions and the KCNE1 mRNA quantity obtained with siRNA scramble.

### EXAMPLES

### Example 1. Use of cationic lipids with cationic moieties for siRNA transfection

### 1.1. Materials and Methods

### 1.1.1. Nucleic acids, cationic lipids and preparation of complexes.

3'-Rhodamine labeled anti-GFP siRNA and the 5'-Rhodamine labeled control siRNA were provided by Qiagen (Chatsworth, CA, USA). Stock. Unlabeled anti-GFP siRNA was provided by Eurogentec (Seraing, Belgium). Anti-GFP siRNAs had for sense sequence: GCAAGCUGACCCUGAAGUUCAU (SEQ ID NO: 1). The Silencer® GAPDH siRNA (human, mouse, rat) targeting the GAPDH mRNA was provided by Ambion^{®} (Cambridgeshire, United Kingdom). Human anti-Lamin A/C siRNA was provided by Santa Cruz Biotechnology (Santa Cruz, CA, USA). The plasmid pTER, encoding anti-GFP siRNA hairpin, was obtained by inserting the modified H1 promoter between the BglII and HindII sites of pCDNA4TO (Invitrogen Life technologies, Carlsbad, CA, USA) and was kindly provided by A. Polesskaya (Villejuif, France). Plasmids were purified from recombinant Escherichia Coli using EndoFree plasmid purification columns (Qiagen, Chatsworth, CA, USA). Lipidic aminoglycoside derivatives and BGTC were synthesized as previously described (2-4.)

3-β-(N-(N',N'-dimethylethane)carbamoyl)cholesterol (DC-Chol) and Dioleoyl phosphatidyl ethanolamine (DOPE) were from Aventi Polar Lipids, Inc (Alabaster, AL, USA). Polyethyleneime (PEI) 25 kDa was from Sigma (St Louis, MO, USA). RNAiFect, X-treme-GENE and Lipofectamine 2000 were respectively from Qiagen (), Roche Diagnostics () and Invitrogen (). Cationic liposomes of lipidic aminoglycoside derivatives/DOPE and BGTC/DOPE were prepared as described in ref. With minor changes, i.e. the dispersion was sonicated for 10 minutes and stored at 4°C. Lipidic aminoglycoside derivatives/nucleic acids complexes and BGTC/nucleic acids lipoplexes were prepared by mixing equal volumes of cationic liposomes at different concentrations, with plasmid DNA or siRNA at the desired concentration in 300 mM NaCl.

### 1.1.2. In vitro transfection.

d2GFP (human lung cancer) cells were cultured in flasks, at 37 °C in 5% CO₂/humidificated atmosphere, in D-MEM with Glucose, 1-Glutamine and Pyruvate supplemented with 1% streptomycin/penicillin, geneticin at 0.8 mg/ml (GIBCO, Invitrogen Life technologies, Carlsbad, CA, USA) and 10% fetal calf serum (Eurobio, Courtaboeuf, France). One day before transfection, cells were transferred onto 24-well culture plates at cell counts of 65000 resulting in approximately 70-80 % confluence 24 hours later. Transfection was performed in each well by adding 50 µl of complexes in 500 µl of serum and geneticin-free D-MEM. After 2h, the transfection medium was replaced by 1 ml of D-MEM containing 10% of fetal calf serum and 1% streptomycin/penicillin. Transfection experiments were performed in triplicate and residual GFP expression was quantified 24 hours after transfection. For the time course experiment, transfection was performed as described above with the following modifications. Transfected cells were transferred every three days onto a new 24-well culture plate with an appropriate cell counts to have 90 % confluence on the day of GFP quantification. Transfection experiments were performed in triplicate and residual GFP expression was measured 1, 2, 3, 5, 6 and 9 days after transfection.

For RNA isolation, cells were placed in 6-well dishes at cell counts of 250000 cells per well. Transfection was performed in each well by adding 100 µl of complex formulation in 1 ml of serum-free and geneticin-free D-MEM. After 2h at 37 °C the transfection medium was replaced by 1 ml of D-MEM containing 10% of fetal calf serum and 1% streptomycin/penicillin. After 24 hours, RNA was isolated.

### 1.1.3. Gene expression and inhibition.

After 24 hours, transfected cells were washed twice with 500 µl of PBS, lysed with Reporter Lysis Buffer (Promega, Madison, WI) supplemented with a protease inhibitor cocktail (Roche Diagnostics, Mannheim, Germany). The complete lysis was ensured by one freezing-thawing (-80°C/20°C). Samples were then centrifuged at 10000 g for 5 min. GFP fluorescence measurements were assayed on a 180 µl aliquot of supernatant and performed on a Victor² (Perkin Elmer, Les Ulis, France) at excitation and emission wavelengths of 485 nm and 535 nm, respectively. Fluorescence was normalized to the total protein concentration of the sample, obtained using a BCA assay kit (Pierce, Rockford, IL, USA). Residual GFP expression was expressed as a percentage of the fluorescence of control sample.

Lamin A/C immunostaining was performed on cells which were washed with PBS and fixed 5 minutes with a PAF 2% solution, then incubated 5 minutes in a PBS-Triton 0,2% solution. First cells were incubated 1 hour with primary anti-Lamin antibody (1/50) (Lamin A/C (H-110), a rabbit polyclonal antibody against 231-340 mapping within an internal region of Lamin A of human origin (Santa Cruz Biotechnology, CA, USA). Then, cells were incubated with the Rabbit IgG-Biotynilated (Sigma, St Louis, MO, USA) (1/800) for 1 hour, and with the Streptavidine-Rhod (1/200) for 30 minutes (Molecular Probes). Cells were observed on a rhodamine excitation using the Dako Fluorescent Mounting Medium (Dako, Cytomacion, Denmark).

### 1.1.4. Fluorescence microscopy.

Fluorescence microscopic examination of transfected cells was carried out using an inverted fluorescence microscopy Axiovert 200 M (CarlZeiss, Gottingen, Germany).

### 1.1.5. Ethidium bromide Fluorescence studies.

Ethidium bromide fluorescence measurements were carried out on Kontron SFM25 spectrofluorimeter at excitation and emission wavelengths of 260 nm and 590 nm, respectively. Fluorescence was monitored immediately after adding ethidium bromide (final concentration 5 µM) to samples prepared at 10 µg/ml of nucleic acid (siRNA or DNA).

### 1.1.6. Dynamic light scattering.

Dynamic light scattering measurements were made on a Zetasizer 300HSA (Malvern, Worcestershire, United Kingdom) at 20°C. Measurements were monitored on samples prepared at 10 µg/ml of nucleic acid containing various amounts of cationic lipid.

### 1.1.7. Cryo-TEM micrography.

siRNA/cationic lipid complexes were prepared at 10 µg siRNA/ml with the appropriate amount of cationic lipid. A 5 µl sample was deposited onto a holey carbon coated copper grid; the excess was blotted with a filter paper, and the grid was plunged into a liquid ethane bath cooled with liquid nitrogen (Leica EM CPC). Specimens were maintained at a temperature of approximately -170 °C, using a cryo holder (Gatan), and were observed with a FEI Tecnai F20 electron microscope operating at 200 kV and at a nominal magnification of 50000X under low-dose conditions. Images were recorded with a 2Kx2K Gatant slow scan CCD camera.

### 1.1.8. Gene expression inhibition on a RNA level.

Total RNA was extracted using TRIzol reagent (Invitrogen, Cergy Pontoise, France), and the Rneasy® Mini Kit (Qiagen, Chatsworth, CA, USA). mRNA was purified using the oligotex® mRNA midi Kit (Qiagen, Chatsworth, CA, USA). Reverse transcription was performed using the SuperScript^{™}III (Invitrogen Life technologies, Carlsbad, CA, USA) as described by the supplier. Real-time quantitative PCR was performed using the ABI Prism^{®} 7900HT sequence detection system (Applied Biosystems) and the data were collected after instrument spectral compensations by the Applied Biosystems SDS 2.1 software. Sequence for GFP reverse primer was 5'-CGGGCATGGCGGACTT-3' (SEQ ID NO:2), for the FAM-probe 5'-CAGCACGACTTCTTC-3' (SEQ ID NO:3) and for the GFP forward primer 5'-GCTACCCCGACCACATGAAG-3' (SEQ ID NO:4). Control DNA-free samples were run for each experiment. The cycling conditions included a hot start at 95 °C for 10 min followed by 40 cycles at 95 °C for 15 s and 60 °C for 1 min. A single amplicon of the appropriate size was detected using gel electrophoresis. The HPRT gene was used for normalizing the data. Values corresponded to the percentage of normalized GFP, or GAPDH, or LaminA/C mRNA amount obtained in cells transfected with anti-GFP or GAPDH or LaminA/C siRNA versus quantity obtained with Control-siRNA.

### 1.2. Results

### 1.2.1. Gene expression inhibition activities of cationic vectors/siRNA complexes.

First, we assessed the potency of cationic vectors, currently used for plasmid DNA transfection, to inhibit GFP expression through the delivery of anti-GFP siRNA molecules in transformed cell line (d2GFP) expressing the cytoplasmic GFP. Anti-GFP siRNA molecules were complexes with BGTC/DOPE or DC-Chol/DOPE or PEI. One day post-transfection, the inhibition of GFP expression was monitored by fluorescence GFP measurement. Figure 1 shows that the residual GFP expression decreased as the cationic vector/siRNA charge ratio, expressed as moles of positive charge/moles of negative charge, increased. The residual GFP expression obtained with siRNA complexed with cationic liposomes of BGTC, DC-Chol and PEI was about32, 52 and 55 %, respectively. These results highlighted the need to develop new vectors with increased efficiency to inhibit gene expression mediated by siRNA. BGTC-DOPE liposomes were selected as standard cationic vector for the comparison of gene expression inhibition in the further experiments.

### 1.2.2. Chemical structure of lipidic aminoglycoside derivatives.

Lipidic aminoglycoside derivatives are amphiphiles with a self-aggregating hydrocarbon tail linked to aminoglycoside headgroup. Figure 2 represents the various lipidic aminoglycoside derivatives used in this study. They consist of tobramycine, kanamycine, paromomycine or neomycine, as cationic headgroup linked to dioleyl chains via a succinyl spacer. Tobramycine and kanamycine belong to the class of 4,6-disubstituted 2-deoxystreptamine ring and paromomycine and neomycine belong to the class of 4,5-disubstituted 2-deoxystreptamine ring.

### 1.2.3. Colloidal stability of cationic liposomes/nucleic acid complexes.

We investigated the physicochemical properties of complexes resulting from the association of DNA or siRNA with cationic liposomes of BGTC or lipidic aminoglycoside derivatives, as a function of the cationic lipid/nucleic acids charge ratio (Fig. 3). To calculate the charge ratio, we assumed that 1 µg of siRNA is 3 nmoles of charged phosphate and that 2, 4, 3, 4 and 6 positive charges were brought by BGTC, DOST, DOSK, DOSP and DOSN respectively. Dynamic light scattering analysis of cationic liposomes complexed with siRNA revealed, as that obtained with DNA and previously reported for lipopolyamine (5), the presence of a three zone model of colloidal stability. The three different zones named A, B and C, are determined by the cationic lipid/nucleic acids charge ratio. In zone A, for low cationic lipid/nucleic acids charge ratios, negatively charged, colloidally stable complexes with partially condensed nucleic acid are formed. Zone B, contains neutrally charged, large and colloidally unstable particles. Zone C, designates for positively charged, small and stable complexes. Most importantly, BGTC-DOPE/siRNA complexes (Fig.3A) exhibited a large zone B with BGTC/siRNA charge ratios ranging from 2 to 8, where complexes flocculated and thus had a mean diameter over 700 nm. By contrast, cationic liposomes of lipidic aminoglycoside derivatives complexed with siRNA (Fig. 3*B-E*) displayed a shorter zone B than with BGTC, and complexes had a mean diameter about 400 nm. In zone C, BGTC-DOPE/siRNA complexes exhibited a mean diameter of 225 nm whereas a mean diameter of 81, 105, 58 and 57 nm was obtained with siRNA complexed with DiOleylamine-A-Succinyl-Tobramycine-DOPE (DOST-DOPE), DiOleylamine-A-Succinyl-Kanamycine-DOPE (DOSK-DOPE), DiOleylamine-A-Succinyl-Paromomycine-DOPE (DOSP-DOPE) and DiOleylamine-A-Succinyl-Neomycine-DOPE (DOSN-DOPE) liposomes, respectively. Therefore, cationic liposome of lipidic aminoglycoside derivatives were able to form small particles when complexed with siRNA molecules. Of note, there were no marked differences in particle size between cationic liposomes of BGTC or lipidic aminoglycoside derivatives when they were formulated with plasmid DNA (Fig.3).

Next, we investigated on the same samples, the siRNA and DNA complexation by ethidium bromide fluorescence measurements. As a general trend, fluorescence intensity decreased as the cationic lipid/nucleic acids charge ratio increased. Fluorescence intensity decreased in zone A from 100% to a value close to zero and stayed at this minimum value in zones B and C. Although, complexation of DNA with either liposomes of BGTC or the various lipidic aminoglycoside derivatives led to similar decreasing of fluorescence intensity, differences were observed with siRNA molecules. The slope of the decreasing of the fluorescence intensity as a function of the charge ratio which varied significantly between lipidic aminoglycoside derivatives was steeper with DOSN and DOSP compared to that observed with DOSK and DOST. By contrast, a lag before the fluorescence intensity decreased was observed with siRNA complexes with BGTC. Agarose gel electropheresis of cationic lipid mixed with DNA and siRNA confirmed results obtained in fluorescence experiments (data not shown).

### 1.2.4. Cryo transmission electron micrographs of cationic lipsomes/siRNA complexes.

BGTC/DOPE vesicle preparation was made of unilamellar liposomes of a diameter comprised in a range of 30-70 nm (Fig. 4A). Mixing BGTC-DOPE liposomes with siRNA solution led to the formation of small compact and round-like structures in a range of 200-500 nm in size (Fig. 4B). These structures were made of a stack of several lipid layers separated by electron dense densities that likely correspond to siRNA molecules. A measured distance of 7.0 nm corresponded to the thickness of the lipid bilayer and the diameter of the double strand siRNA molecules. With well-oriented condensed structure, fine striations with a 3.0 nm spacing were visible that likely revealing a regular arrangement between two lipid membranes (Fig. 4C).

With DOST-DOPE and DOSK-DOPE liposomes, small complexes in a range of 100-300 nm in size were induced in the presence of siRNA molecules (Fig. 4D-G). They formed "onion-like" structures made of a regular superimposition of lipid bilayer and siRNA molecules and strongly resembled those formed with BGTC-DOPE liposomes. A typical lipoplexe (Fig. 4D and detailed Fig. 4E) revealed a 6.7 nm spacing between two repeat layers, the electron dense layer corresponding to the layer of siRNA molecules. It is worth to note that siRNA molecules appeared well-ordered on the lipid membrane as shown on the edge of the lipoplexe (upper right) with the same spacing measured on BGTC-DOPE complexes.

With DOSP-DOPE and DOSN-DOPE liposomes, complexes in a range of 50-300 nm in size were formed after addition of siRNA molecules (Fig. 4H-K). And the complexes formed with DOSN-DOPE liposomes exhibit a tendency to form grape of small condensed structures. Unlike the complexes formed with the three previous cationic lipids, the complexes made with DOSP-DOPE and DOSN-DOPE liposomes possessed a more irregular structure. While they were composed of a super-imposition of lipid bilayer and siRNA molecule, this arrangement did not extend over a long distance. One explanation of this difference could be attributed to the size of the liposomes (diameter range 30-50 nm). They had a smaller size than those made with three other cationic lipids (data not shown) and could not induced the formation of the onion-like structure.

It is important to mention that when the liposomes made with the five different lipid composition exposed to 300 mM NaCl, the small liposomes remain unilamellar vesicle while the large one became double lipid bilayer liposomes due to an osmotic effect (6 and data not shown).

### 1.2.5. Gene expression inhibition activities of lipidic aminoglycoside derivatives/siRNA complexes.

Figure 5A shows that GFP fluorescence level of d2-GFP transfected cells with BGTC-DOPE/siRNA complexes decreased progressively as the BGTC/siRNA charge ratio increased. By contrast, DOST-DOPE/siRNA complexes led to sharp decrease of GFP fluorescence to 11% for a DOST/siRNA charge ratio of 4 and stayed at this minimal value for higher charge ratios. Surprisingly DOSK-DOPE/siRNA complexes led to a progressive decrease in GFP fluorescence and a minimal fluorescence level of 41% was reached for a DOSK/siRNA charge ratio of 12. Both aminoglycoside derivatives from the 4,5-disubstituted class, DOSP-DOPE and DOSN-DOPE, led to a progressive decrease of GFP expression as a function of the cationic lipid/siRNA charge ratio, and the minimal residual fluorescence level of 10% was reached for a cationic lipid/siRNA charge ratio of 8. Transfection of a control siRNA in the same conditions with the various cationic lipids did not affect the GFP expression compared to non-transfected cells (data not shown). For the further experiments, we selected the cationic lipid/siRNA charge ratios of 4 for siRNA complexed with BGTC and DOST, 10 with DOSP and 12 with DOSN and DOSK.

Figure 5B illustrates the influence of the cationic lipid on the GFP expression as a function of the amount of transfected siRNA. For BGTC-DOPE and DOSK-DOPE liposomes, GFP expression decreased progressively and minimal GFP expression of 50% was obtained for 500 ng of siRNA. By contrast, cationic liposomes of DOSN-DOPE, DOST-DOPE and DOSP-DOPE led to a minimal GFP expression of 20% at 300 ng of siRNA per well. Figure 5C shows, on the same experiment the visualization of GFP expression inhibition by DOSP-DOPE cationic liposomes which led to a strong reduction in GFP fluorescence level probably related to the high siRNA internalization molecules as visualized by rhodamine fluorescence (Fig. 5C). However, DOSK-DOPE cationic liposomes led also to siRNA cell internalization but the GFP fluorescence was slightly reduced compared to that obtained with siRNA control (Fig. 5C). As control, naked siRNA was assessed for GFP inhibition but failed to decrease GFP fluorescence level (Fig. 5C). BGTC-DOPE cationic liposomes led to an intermediate GFP expression inhibition between that obtained with DOSK and DOSP.

Next, we performed RNAi experiments with siRNA targeting the endogenous Lamin A/C expression. Here, RT-PCR results indicated that d2-GFP cells transfected with DOSP/siRNA complexes had very little residual Lamin A/C mRNA when compared with cells transfected with control siRNA (Fig. 5D), a finding demonstrating that DOSP/siRNA complexes can also allow the efficient knock-down of the expression of an endogeneous gene in d2GFP cells. To broaden our conclusions, we also found that DOSP/anti-lamin A/C siRNA complexes were highly efficient for silencing of Lamin A/C expression in other human cells lines such as HEK293 cells (human embryonic kidney cells) and HeLa cells (derived from a human epithelioid cervical cancer), a very low level of residual Lamin A/C mRNA being again observed (Fig. 5D).

Experiments were also performed using the aminoglycoside derivatives according to the invention for transfecting siRNA, without the use of a neutral lipid adjuvant such as DOPE.

The GFP-expressing human lung cancer H1299 cells were transfected with control siRNA or 3'-rhodamine labeled anti-GFP siRNA. The siRNA molecules were formulated in the absence ("naked" siRNA) or in the presence of DOSP, but in any case without DOPE. The transfected H1299 cells were observed using a FITC filter to visualize GFP fluorescence or a rhodamine filter to visualize siRNA internalization.

Results presented in Figure 6A show that delivery of anti-GFP siRNA molecules into H1299 cells by DOSP/siRNA complexes, without the use of a neutral lipid adjuvant such as DOPE, led to a strong reduction of the number of GFP-positive cells (represented by white/light grey areas) (panel D) which correlated with a high cellular uptake of the siRNA molecules (as visualized in panel F by the fluorescence due to the rhodamine-labelled anti-GFP siRNA, see grey areas). Naked "unreacted" siRNA was used as control of the efficiency of the ICAFectin DOSP for siRNA delivery. Panels A, C and E show that there was neither suppression of GFP expression nor siRNA uptake when using naked "unreacted" siRNA, a finding confirming the role of the DOSP reagent for siRNA delivery.

In addition, Lamin A/C silencing efficiency using DOSP reagent complexed with anti-Lamin A/C siRNA, but without a neutral lipid adjuvant, has also been tested by RT-PCR. Anti-LaminA/C siRNA amounts ranging from 3.75 to 200 ng/well were formulated with DOSP.

Results of real-time quantitative RT-PCR analysis of human Lamin A/C mRNA after transfection of HeLa cells displayed in Figure 6B show that DOSP reagent is efficient with very low amount of siRNA (normalization to HPRT1).

Finally, the efficiency of DOSP reagent was compared with that of commercial reagent HiPerFect for transfecting anti-LaminA/C siRNA. Anti-LaminA/C siRNA at 18.75 and 37.5 ng/well were formulated with DOSP or the commercial reagents HiPerFect.

Results of real-time quantitative RT-PCR analysis of human Lamin A/C mRNA after transfection of HeLa cells displayed in Figure 6C show that DOSP reagent was more efficient than HiPerFect (normalization to HPRT1).

### 1.2.6. Gene expression inhibition activities of lipidic paromomycine derivatives/siRNA complexes in comparison with commercial reagents.

Figure 7 indicates that DOSP-DOPE led to a higher decrease in GFP fluorescence level in d2GFP transfected cells compared to that observed with the X-tremeGene and the Lipofectamine^{™} 2000. Results were normalized with the residual GFP expression obtained with DOSP-DOPE /siRNA complexes. No significant difference was observed between the DOSP-DOPE and the RNAiFect^{™} reagent.

### 1.2.7. Kinetics of GFP expression inhibition

Next, we investigated the influence of cationic liposomes/siRNA complexes on GFP expression as a function of time (Fig. 8). For transfected cells with siRNA complexed with DOSN-DOPE or DOSP-DOPE, the residual GFP expression was about 10% for 5 days then increased progressively and returned to 100% at day 9. Cells transfected with DOST-DOPE/siRNA complexes displayed the same kinetic of GFP expression but the residual GFP expression was about 20%. Figure 8 also indicates that DOSK-DOPE liposomes led to the maximum of GFP fluorescence reduction at day 2.

### 1.2.8. In vitro transfection of plasmid by amino-glycosides derivatives and BGTC-DOPE liposomes

We assessed the potential of aminoglycosides derivatives to deliver not only siRNA but also plasmid DNA encoding small hairping RNA against GFP into d2GFP cells (Fig. 9). The results showed that GFP expression decreased as the cationic lipid/DNA charge ratio increased reaching a minimal fluorescence value depending of the cationic lipid. Except for DOSN-DOPE/DNA complexes increasing the cationic lipid/DNA charge ratio led to an increase of the GFP expression. These results are in good agreement with those obtained with the transfection of a plasmid encoding luciferase, indicating that the GFP expression inhibition was correlated with the transfection efficiency.

We also assessed the potential of aminoglycosides to co-deliver a plasmid encoding KCNE1 and the siRNA targeted against KCNE1 mRNA. Figure 9B shows that DOSP-DOPE and DOSN-DOPE led to the smallest level of KCNE1 mRNA. Figure 9D shows that the inhibition of the plasmid co-injected with the siRNA was function of the amount of siRNA transfected.

### 1.3. Conclusions

The colloidal stability of lipidic aminoglycoside derivatives/siRNA complexes, which depended on the charge ratio, determined three main zones-A, B and C- which corresponded to negatively, neutrally and positively charged complexes respectively. This colloidal stability behavior was previously observed with complexes resulting from association of lipopolyamine micelles (5), bisguanidinium liposomes (7, and this study) or DOTAP liposomes with DNA molecules. However, while the zone B was similar with DNA complexed with the newly synthetized lipidic aminoglycoside derivatives, it was reduced upon complexation with siRNA.

In addition, the mean diameter of lipid aminoglocoside derivatives/siRNA complexes in the three zones was smaller than that measured with lipidic aminoglycoside derivatives/DNA complexes. In Zone C, the mean diameter was about 81, 105, 58 and 57 nm for complexes resulting from the association of siRNA with DOST, DOSK, DOSP and DOSN, respectively.

Among lipidic aminoglycoside derivatives, the boundary between zone A and B was shifted toward a decreased charge ratio when siRNA was complexed with 4,5-disubstituted 2-deoxystreptamine ring aminoglycoside compared to 4,6-disubstituted 2-deoxystreptamine ring aminoglycoside and BGTC.

In addition, although regular spacing about 6.7 nm were observed in all cases, the morphology of the complexes differed with the two different classes of aminoglycosides: complexes with 4,6-disubstituted 2-deoxystreptamine ring aminoglycoside and BGTC displayed classical "onion-like" structures made of a regular superimposition of lipid bilayer and siRNA molecules, while complexes with 4,5-disubstituted 2-deoxystreptamine ring aminoglycoside surprisingly formed distinct structures with grape of small condensed structures.

The difference in morphology of complexes with the two classes of aminoglycoside may not be fully explained at the present time. However, it might be related to the geometry of the lipidic derivatives, assuming that 2-deoxystreptamine 4,6 disubstituted and 2-deoxystreptamine 4,5 disubstituted could adopt a cylindrical and conical shape, respectively. This might impact the formation and the size of the cationic liposmes. It has been shown that concentric multilamellar organization over long distance was more easily formed with large initial liposomes able to disrupt and reassemble sandwiching DNA molecules. On the other hand, small liposomes are like micelles leading to ordered lamellar microdomains.

In any case, the shift in the boundary between zones A and B and the difference in complex structure between 4,5-disubstituted 2-deoxystreptamine ring aminoglycoside and 4,6-disubstituted 2-deoxystreptamine ring aminoglycoside was correlated with the higher efficiency of complexes with ,5-disubstituted 2-deoxystreptamine ring aminoglycoside to induce functional interference in mammalian cells. Indeed, highly effective complexes for gene silencing were obtained in zone C with paromomycine headgroup characterized by their small diameter (57 nm) and high colloidal stability.

It is known that cationic lipid/nucleic acid complexes are internalized into cells through an endocytosis process mediated by electrostatic interactions between positively charged complexes and the cell membrane. Endosomal escape is recognized as one of the main limiting steps of the currently cationic reagents. Although no certain explanation for the particularly high efficiency of these complexes is available, it is possible that complexes of lipidic 4,5-disubstituted 2-deoxystreptamine ring aminoglycoside derivatives/siRNA could improve RNA releasing in the cell cytoplasm because of the specific physicochemical properties of the aminoglycoside/siRNA self assemblies such as small size and the structural features and the increase flip-flop mechanism due to the flexibility of the aminoglycoside capable to match with the negative charge density of the leaflet membrane. The particular morphology obtained with the 4,5 disubstituted 2-deoxystrepatmine ring should led to more labile supramolecular structures compared to those obtained with the 4,6 disubstituted 2-deoxystrepatmine ring conferring in this condition a more facilitated release of siRNA which is a crucial step for their incorporation into RISC complexes and the subsequent gene expression inhibition.

The obtained results also indicate that the tranfecting compounds with 4,5-disubstituted 2-deoxystreptamine ring aminoglycoside are even more efficient than commercially available transfection systems.

Globally, the inventors have thus generated and characterized transfecting compounds specially adapted to siRNA transfection.

### BIBLIOGRAPHY

1. US 2003-0054556
2. Sainlos M., Hauchecorne M., Oudrhiri N., Zertal-Zidani S., Aissaoui A., Vigneron JP., Lehn JM., Lehn P. Kanamycin A-derived cationic lipids as vectors for gene transfection. Chembiochem. 2005 Jun;6(6):1023-33;
3. Sainlos M., Transfert de genes a l'aide de substances bioactives. These de doctorat de l'université de Paris 6, 6 Juillet 2004 ;
4. Belmont P, Aissaoui A, Hauchecorne M, Oudrhiri N, Petit L, Vigneron JP, Lehn JM, Lehn P. Aminoglycoside-derived cationic lipids as efficient vectors for gene transfection in vitro and in vivo. J Gene Med. 2002 Sep-Oct;4(5):517-26
5. Pitard, B., Aguerre, O., Airiau, M., Lachagès, A.M., Boukhnikachvili, T., Byk, G., Dubertret, C., Herviou, C., Scherman, D., Mayaux, J.F. and Crouzet, J. Virus-sized self-assembling lamellar complexes between plasmid DNA and cationic micelles promote gene transfer. Proc.Natl Acad Sci USA (1997) 94, 14412-14417
6. Pitard, B., Oudrhiri, N., Lambert, O., Vivien, E., Masson, C., Wetzer, B., Hauchecorne, M., Scherman, D., Rigaud, J.L., Vigneron, J.P., Lehn, J.M., and Lehn, P. Sterically stabilized BGTC-based lipoplexes : structural features and gene transfection into the mouse airways in vivo. J. Gene Medecine (2001) 3, 478-487
7. Pitard, B., Oudrhiri, N., Vigneron, J.P., Hauchecorne, M., Aguerre, O., Toury, R., Airiau, M., Ramasawmy, R., Scherman, D., Crouzet, J., Lehn, J.M. and Lehn, P. Structural characteristics of supramolecular assemblies formed by guanidinium-cholesterol reagents for gene transfection. Proc. Natl. Acad. Sci. USA (1999) 92, 2621-2626
8. WO2005/014837

## Claims

1. A composition comprising a siRNA and a transfecting compound of general formula (I):
A-Y-L (I),
wherein
• A represents an aminoglycoside of the class of 4,5-disubstituted 2-deoxystreptamine ring,
• Y represents a spacer, and
• L represents:
- either a radical -(R1)R2, wherein R1 and R2 represent, independently of one another, a hydrogen atom or a fatty aliphatic chain or R1 or R2 is absent, with the proviso that at least one of R1 and R2 represents a fatty aliphatic chain,
- or a radical -N-R3 or -O-R3, wherein R3 represents a steroidal derivative
or a salt, an isomer or a mixture thereof.

2. The composition according to claim 1, wherein said aminoglycoside A of the class of 4,5-disubstituted 2-deoxystreptamine ring is selected from paromomycin, neomycin, and ribostamycin.

3. The composition according to claim 1 or 2, wherein said spacer Y comprises at least one chemical function chosen from alkyls having 1 to 6 carbon atoms, ketone functions, ester functions, ether functions, amino functions, amide functions, amidine functions, carbamate functions, thiocarbamate functions, glycerol, urea, thiourea, and aromatic rings.

4. The composition according to claim 3, wherein said spacer Y is selected from radicals of formula:
-C(O)-;
-NH-C(O)-CH₂-CH₂-;
-W-(CH₂-)ₖ-W'-;
and
-(CH₂-)ᵢ-W-(CH₂)ⱼ-
in which i, j and k are integers chosen between 1 and 6 inclusive, and W and W' are groups, which may be identical or different, chosen from ketone functions, ester functions, ether functions, amino functions, amide functions, amidine functions, carbamate functions, thiocarbamate functions, glycerol, urea, thiourea, and aromatic rings.

5. The composition according to any of claims 1-4, wherein L represents the radical -(R1)R2 and the at least one fatty aliphatic chain is selected from saturated or unsaturated alkyl radicals containing 10 to 22 carbon atoms, preferably L is selected from dimyristoyl, dioleyl, and distearyl.

6. The composition according to claim 1, wherein said transfecting compound is selected from :
dioleylamine-A-succinyl-neomycine ("DOSN") of formula: 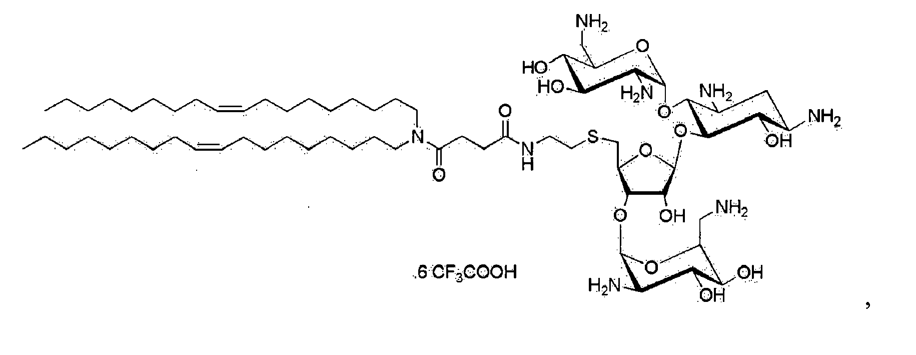
dioleylamine-A-succinyl-paromomycine ("DOSP") of formula: 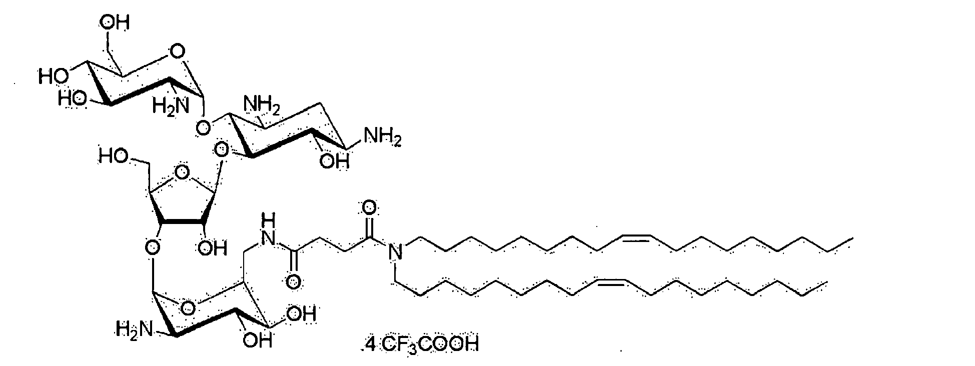
NeoChol of formula: 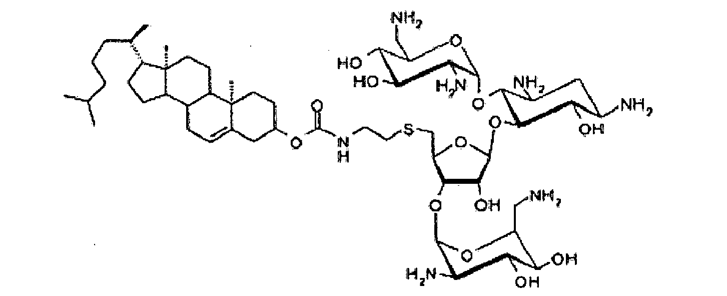
NeoSucChol of formula: 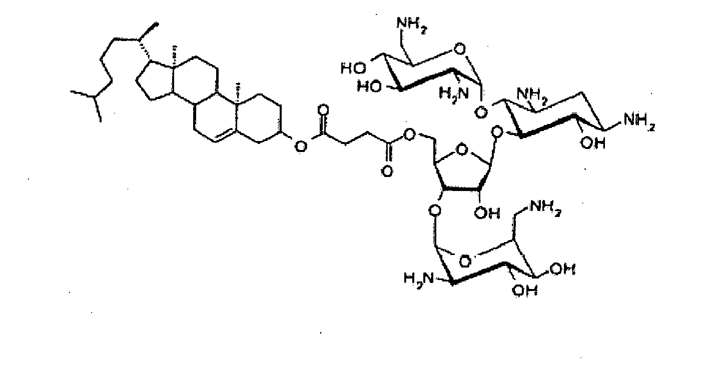
ParomoChol of formula: 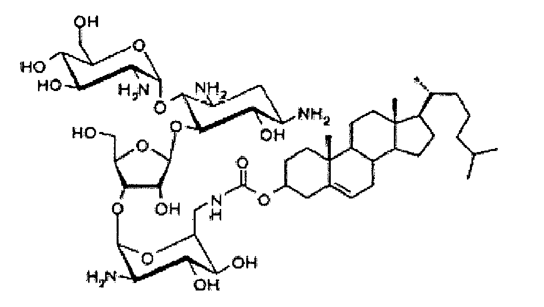
ParomoCapSucDOLA of formula: 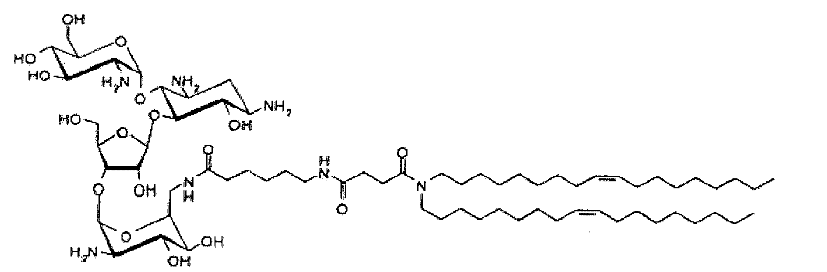
ParomoLysSucDOLA of formula: 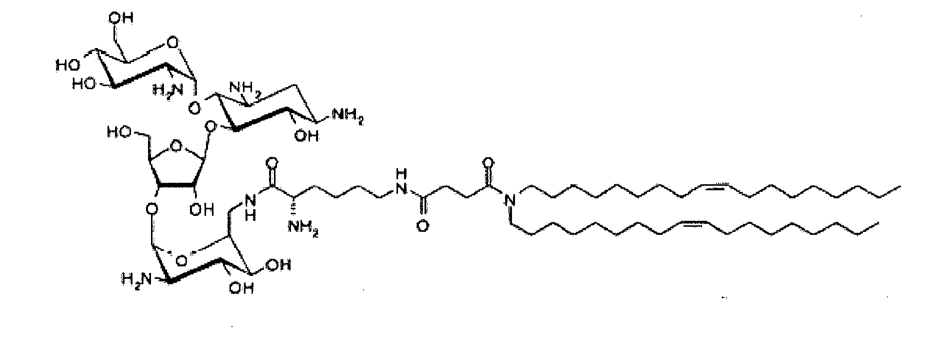
NeodiSucDODA of formula: 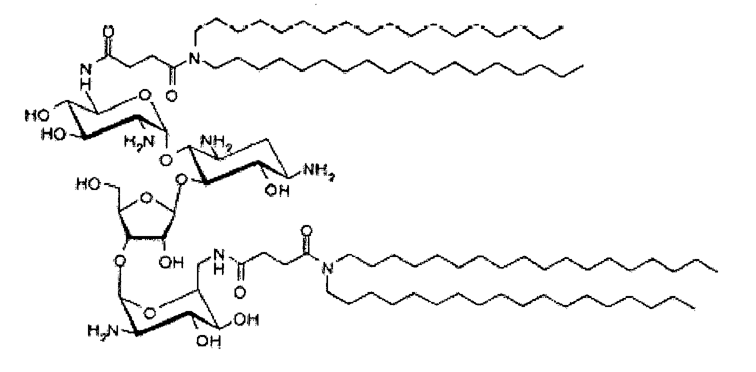
NeodiLysSucDOLA of formula: 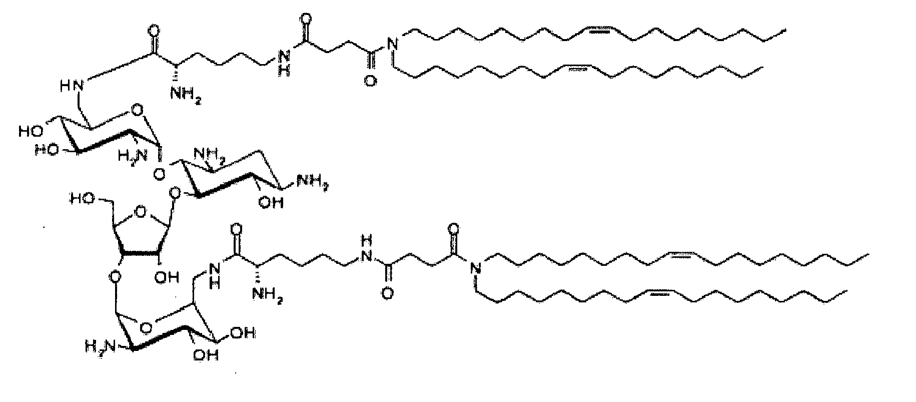
and [ParomoLys]₂-Glu-Lys-[SucDOLA]₂ of formula:

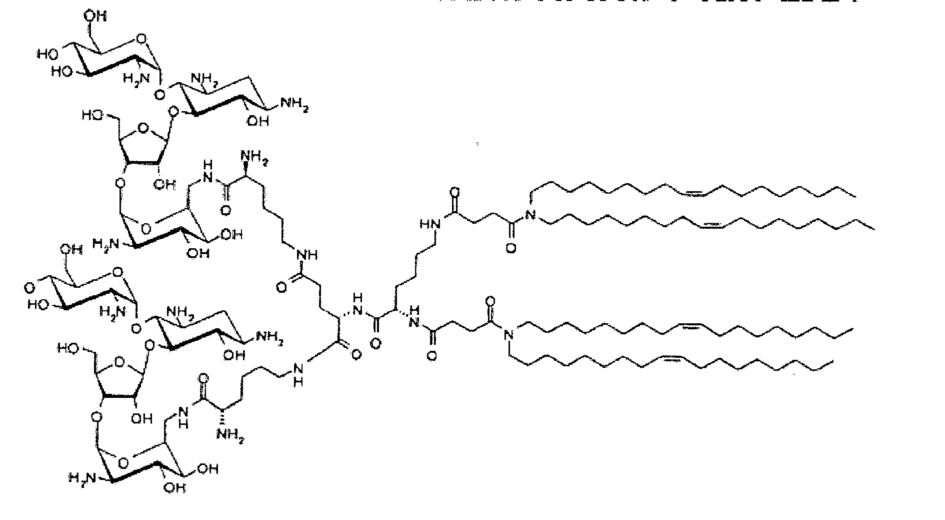

7. The composition according to any of claims 1-6, further comprising at least one adjuvant, preferably at least one of a lipid, peptide, protein, and polymer.

8. The composition according to claim 7, wherein said at least one adjuvant is chosen from neutral lipids, preferably chosen from natural and synthetic lipids which are zwitterionic or lack ionic charge under physiological conditions.

9. The composition according to claim 8, wherein said neutral lipid is chosen from dioleoylphosphatidylethanolamine, oleoylpalmitoylphosphatidyl- ethanolamine, distearoyl-, dipalmitoyl- and dimirystoylphosphatidylethanol- amines and derivatives thereof that are N-methylated 1 to 3 times, phosphatidylglycerols, diacylglycerols, glycosyldiacylglycerols, cerebrosides, sphingolipids, asialogangliosides, dioleoylphosphatidylcholine, and cholesterol.

10. The composition according to any of claims 1-9, further comprising an extracellular or intracellular targeting element, preferably chosen from sugars, peptides, proteins, oligonucleotides, lipids, neuromediators, hormones, vitamins, and derivatives thereof.

11. The composition according to claim 10, wherein said targeting element is covalently attached either to the transfecting compound according to claim 1, or to the siRNA.

12. The composition according to any of claims 1-11, further comprising a vehicle that is pharmaceutically acceptable for an injectable formulation.

13. The composition according to any of claims 1-11, further comprising a vehicle that is pharmaceutically acceptable for administration on the skin and/or mucous membranes.

14. The composition according to any of claims 1-13, for use as a medicinal product.

15. A method for transferring a siRNA into cells in vitro, comprising:
(1) providing a composition according to any of claims 1-13, and
(2) bringing the cells into contact with said composition.

## Patentansprüche

1. Zusammensetzung, umfassend eine siRNA und eine Transfektionsverbindung der allgemeinen Formel (I):
A - Y - L (I),
wobei
• A ein Aminoglykosid aus der Klasse 4,5-disubstutuierter 2-Desoxystreptamin-Ringe darstellt,
• Y einen Spacer darstellt, und
• L
- entweder einen Rest -(R1)(R2) darstellt, wobei R1 and R2 unabhängig voneinander ein Wasserstoffatom oder eine aliphatische Fettkette darstellen, oder R1 oder R2 abwesend ist, unter der Voraussetzung, dass zumindest einer von R1 und R2 eine aliphatische Fettkette darstellt,
- oder einen Rest -N-R3 oder -O-R3 darstellt, wobei R3 ein steroidales Derivat darstellt,
oder ein Salz, ein Isomer oder eine Mischung davon.

2. Zusammensetzung gemäß Anspruch 1, wobei das Aminoglycosid A aus der Klasse 4,5-disubstutuierter 2-Desoxystreptamin-Ringe aus Paromomycin, Neomycin und Ribostamycin ausgewählt ist.

3. Zusammensetzung gemäß Anspruch 1 oder 2, wobei der Spacer Y wenigstens eine chemische Funktion umfasst, die aus Alkylen mit 1 bis 6 Kohlenstoffatomen, Ketonfunktionen, Esterfunktionen, Etherfunktionen, Aminofunktionen, Amidfunktionen, Amidinfunktionen, Carbamatfunktionen, Thiocarbamatfunktionen, Glycerin, Harnstoff, Thioharnstoff und aromatischen Ringen ausgewählt ist.

4. Zusammensetzung gemäß Anspruch 3, wobei der Spacer Y aus Resten der Formel:
-C(O)-;
-NH-C(O)-CH₂-CH₂-;
-W-(CH₂)ₖ-W'-;
und
-(CH₂-)ᵢ-W-(CH₂)ⱼ-
ausgewählt ist, wobei i, j und k ganze Zahlen sind, die aus 1 bis einschließlich 6 ausgewählt sind, und W und W' Gruppen sind, welche identisch oder verschieden sein können und aus Ketonfunktionen, Esterfunktionen, Etherfunktionen, Aminofunktionen, Amidfunktionen, Amidinfunktionen, Carbamatfunktionen, Thiocarbamatfunktionen, Glycerin, Harnstoff, Thioharnstoff und aromatischen Ringen ausgewählt sind.

5. Zusammensetzung gemäß irgendeinem der Ansprüche 1 bis 4, wobei L den Rest -(R1)R2 darstellt, und die wenigstens eine aliphatische Fettkette aus gesättigten oder ungesättigten Alkylresten, welche 10 bis 22 Kohlenstoffatome enthalten, ausgewählt ist, und bevorzugt L aus Dimyristoyl, Dioleyl und Distearyl ausgewählt ist.

6. Zusammensetzung gemäß Anspruch 1, wobei die Transfektionsverbindung ausgewählt ist aus:
Dioleylamin-A-succinyl-Neomycin ("DOSN") der Formel: 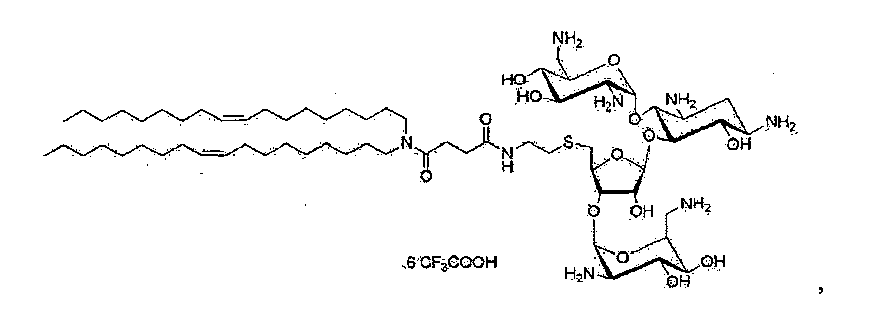
Dioleylamin-A-succinyl-Paromomycin ("DOSP") der Formel: 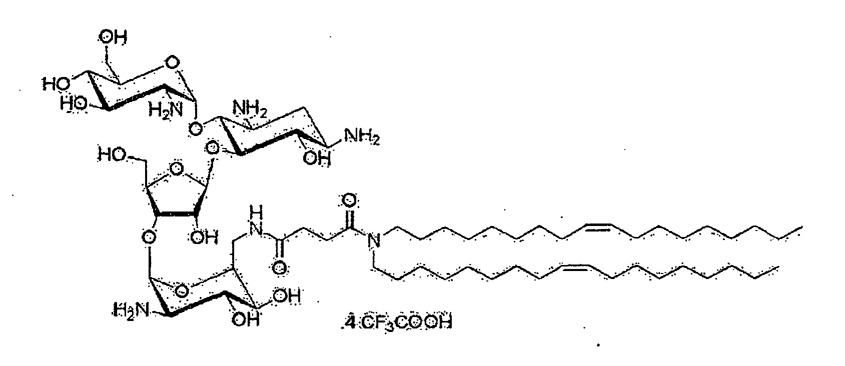
NeoChol der Formel: 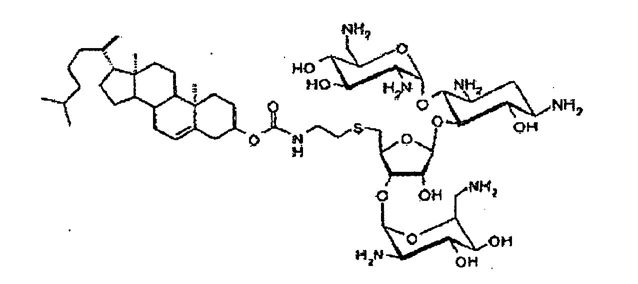
NeoSucChol der Formel: 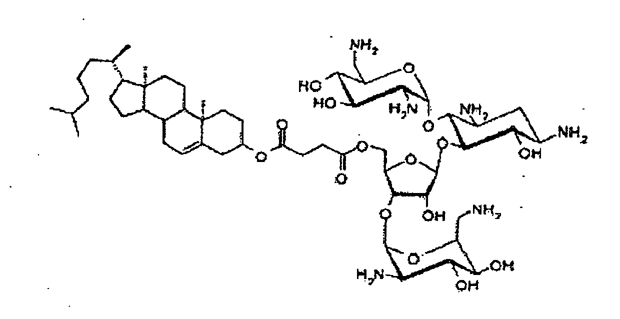
ParomoChol der Formel: 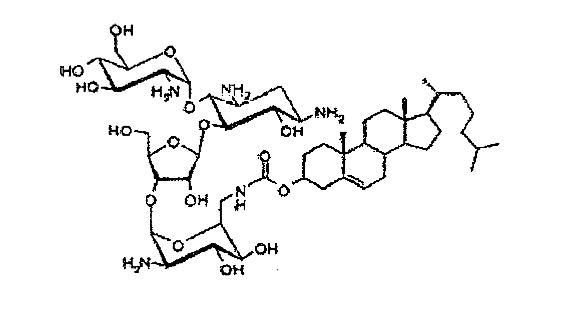
ParomoCapSucDOLA der Formel: 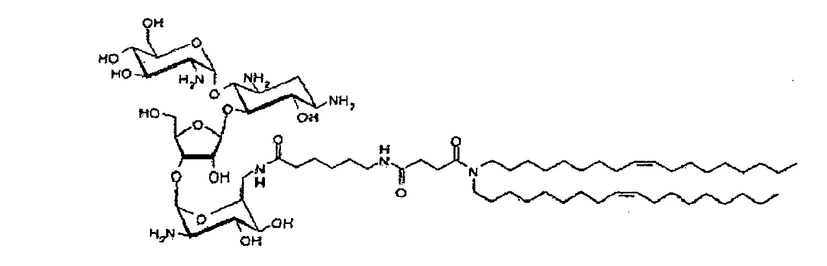
ParomoLysSucDOLA der Formel: 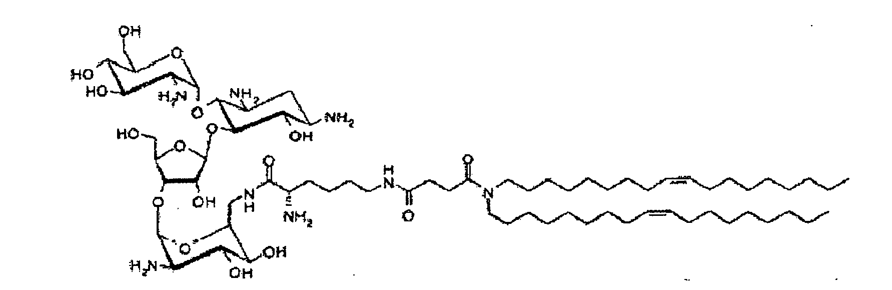
NeodiSucDODA der Formel: 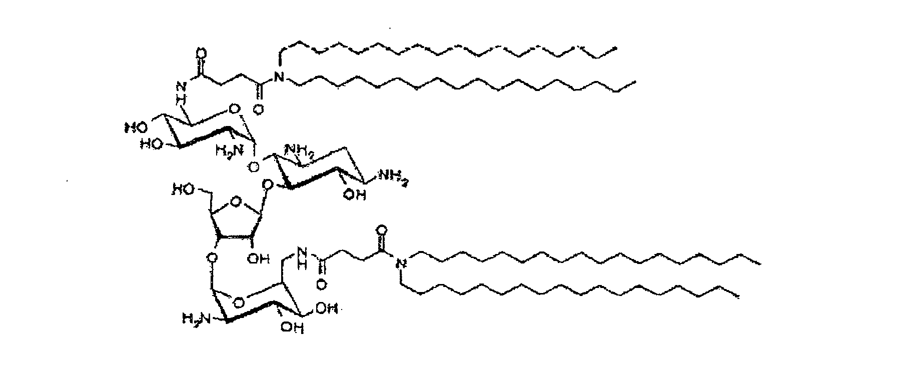
NeodiLysSucDOLA der Fomel: 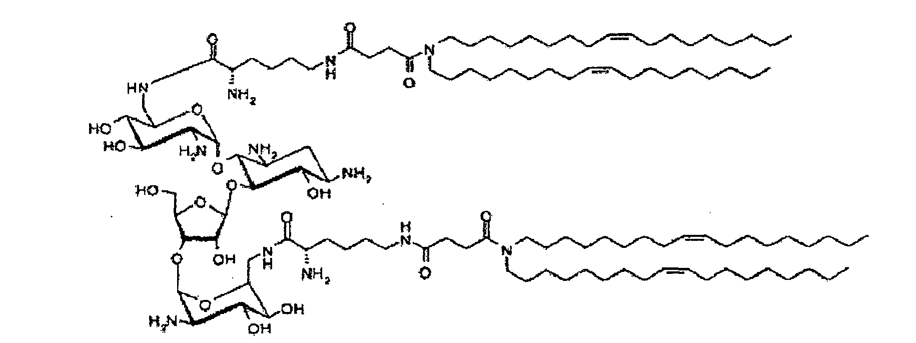
und [ParomoLys]₂-Glu-Lys-[SucDOLA]₂ der Formel:

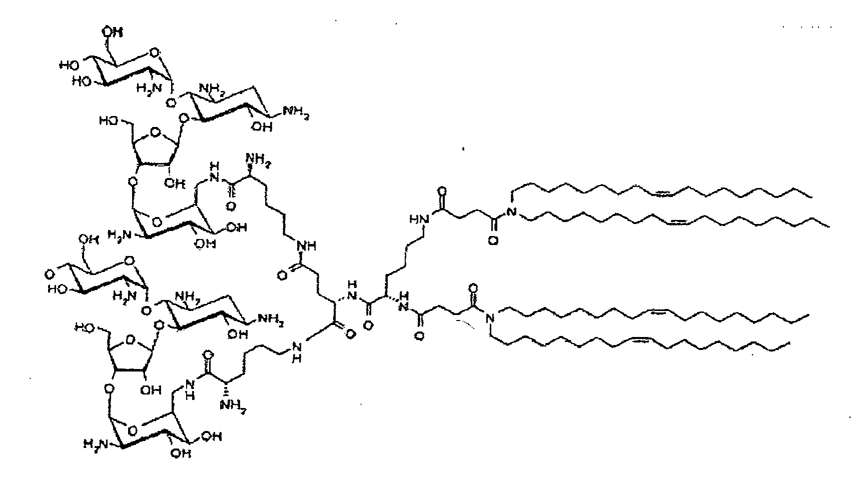

7. Zusammensetzung gemäß irgendeinem der Ansprüche 1 bis 6, welche ferner wenigstens ein Adjuvans, bevorzugt wenigstens ein Lipid, Peptid, Protein und/oder Polymer, umfasst.

8. Zusammensetzung gemäß Anspruch 7, wobei das wenigstens eine Adjuvans aus neutralen Lipiden ausgewählt ist, bevorzugt aus natürlichen und synthetischen Lipiden ausgewählt ist, welche zwitterionisch sind oder unter physiologischen Bedingungen keine ionische Ladung aufweisen.

9. Zusammensetzung gemäß Anspruch 8, wobei das neutrale Lipid aus Dioleylphosphatidylethanolamin, Oleoylpalmitoylphosphatidyl-Ethanolamin, Distearoyl-, Dipalmitoyl- und Dimyristoylphosphatidylethanol-Aminen und Derivaten davon, welche 1- bis 3-fach N-methyliert sind, Phosphatidylglycerinen, Diacylglycerinen, Glycosyldiacylglycerinen, Cerebrosiden, Sphingolipiden, Asialogangliosiden, Dioleylphosphatidylcholin und Cholesterin ausgewählt ist.

10. Zusammensetzung gemäß irgendeinem der Ansprüche 1 bis 9, welche ferner ein extrazelluläres oder intrazelluläres Zielrichtungs-("Targeting-")element umfasst, welches bevorzugt aus Zuckern, Peptiden, Proteinen, Oligonukleotiden, Lipiden, Neuromediatoren, Hormonen, Vitaminen und Derivaten davon ausgewählt ist.

11. Zusammensetzung gemäß Anspruch 10, wobei das Zielrichtungselement entweder an die Transfektionsverbindung gemäß Anspruch 1 oder die siRNA kovalent gebunden ist.

12. Zusammensetzung gemäß irgendeinem der Ansprüche 1 bis 11, welche ferner eine für eine injizierbare Formulierung pharmazeutisch akzeptable Trägersubstanz umfasst.

13. Zusammensetzung gemäß irgendeinem der Ansprüche 1 bis 11, welche ferner eine für eine Verabreichung auf der Haut und/oder den Schleimhäuten pharmazeutisch akzeptable Trägersubstanz umfasst.

14. Zusammensetzung gemäß irgendeinem der Ansprüche 1 bis 13 zur Verwendung als medizinisches Produkt.

15. Verfahren zur Einführung einer siRNA in Zellen *in vitro,* umfassend:
(1) Bereitstellen einer Zusammensetzung gemäß irgendeinem der Ansprüche 1 bis 13, und
(2) In-Kontakt-Bringen der Zellen mit der Zusammensetzung.

## Revendications

1. Composition comprenant un siARN et un composé de transfection de formule générale (I) :
A-Y-L (I),
où
- A représente un aminoglycoside de la classe à cycle de 2-désoxystreptamine 4,5-disubstituée,
- Y représente un espaceur, et
- L représente :
- soit un radical -(R1)R2, où R1 et R2 représentent, indépendamment l'un de l'autre, un atome d'hydrogène ou une chaîne aliphatique grasse ou R1 ou R2 est absent, à condition qu'au moins l'un de R1 et R2 représente une chaîne aliphatique grasse,
- soit un radical -N-R3 ou -O-R3, où R3 représente un dérivé stéroïdien ou l'un de ses sels, isomères ou mélanges.

2. Composition selon la revendication 1, dans laquelle ledit aminoglycoside A de la classe à cycle de 2-désoxystreptamine 4,5-disubstituée est choisi parmi la paromomycine, la néomycine et la ribostamycine.

3. Composition selon la revendication 1 ou 2, dans laquelle ledit espaceur Y comprend au moins une fonction chimique choisie parmi des alkyles comportant 1 à 6 atomes de carbone, des fonctions cétone, des fonctions ester, des fonctions éther, des fonctions amino, des fonctions amide, des fonctions amidine, des fonctions carbamate, des fonctions thiocarbamate, le glycérol, l'urée, la thiourée, et des cycles aromatiques.

4. Composition selon la revendication 3, dans laquelle ledit espaceur Y est choisi parmi les radicaux de formule :
-C(O)- ;
-NH-C(O)-CH₂-CH₂- ;
-W-(CH₂-)ₖ-W'- ;
et
-(CH₂-)ᵢ-W-(CH₂)ⱼ-
dans lesquels i, j et k sont des nombres entiers choisis entre 1 et 6 compris, et W et W' sont des groupes, qui peuvent être identiques ou différents, choisis parmi des fonctions cétone, des fonctions ester, des fonctions éther, des fonctions amino, des fonctions amide, des fonctions amidine, des fonctions carbamate, des fonctions thiocarbamate, le glycérol, l'urée, la thiourée, et des cycles aromatiques.

5. Composition selon l'une quelconque des revendications 1 à 4, dans laquelle L représente le radical -(R1)R2 et la au moins une chaîne aliphatique grasse est choisie parmi des radicaux alkyle saturés ou insaturés contenant 10 à 22 atomes de carbone, de préférence L est choisi parmi les radicaux dimyristoyle, dioléyle et distéaryle.

6. Composition selon la revendication 1, dans laquelle ledit composé de transfection est choisi parmi :
la dioléylamine-A-succinyl-néomycine (« DOSN ») de formule : 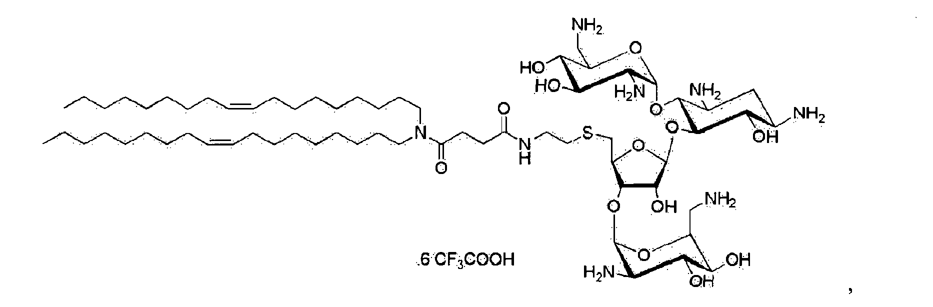
la dioléylamine-A-succinyl-paromomycine (« DOSP ») de formule : 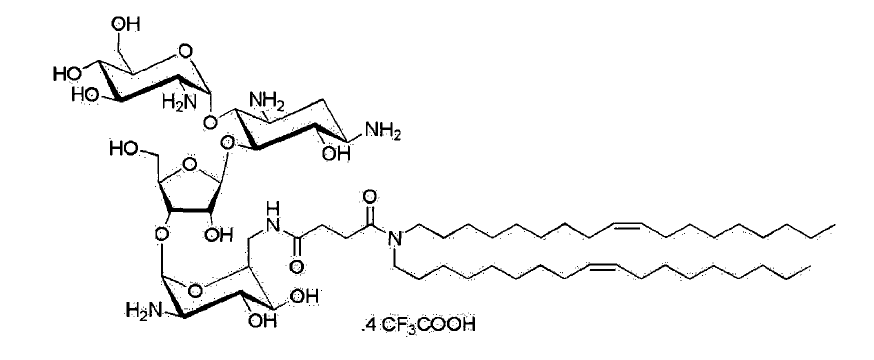
NeoChol de formule : 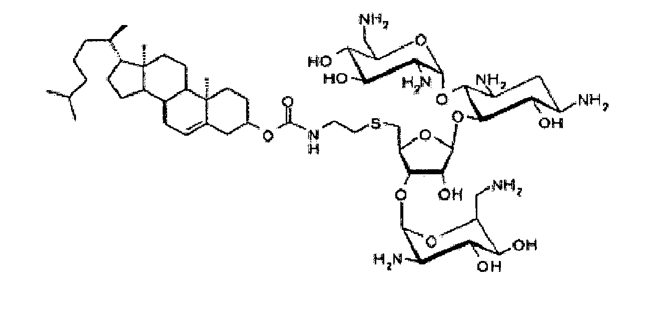
NeoSucChol de formule : 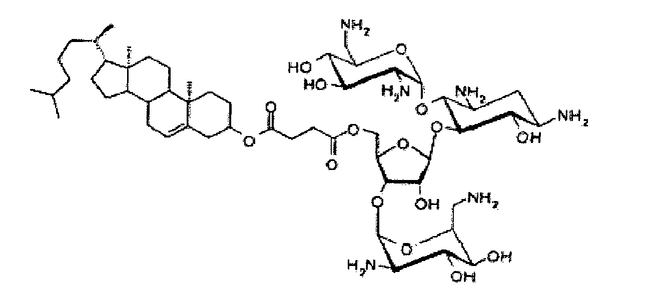
ParomoChol de formule : 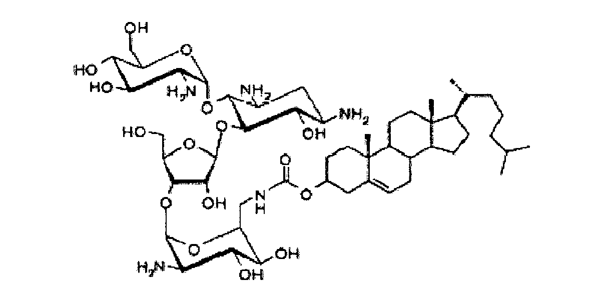
ParomoCapSucDOLA de formule : 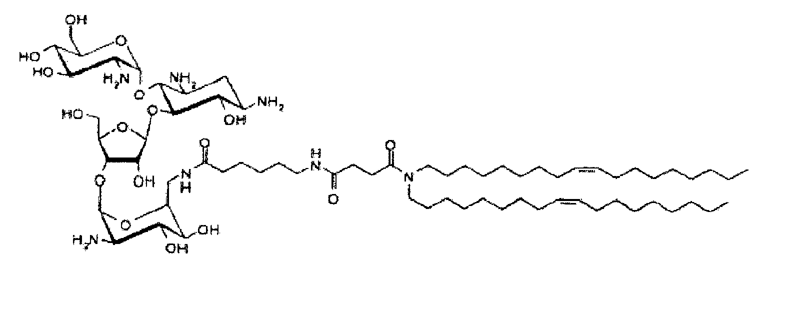
ParomoLysSucDOLA de formule : 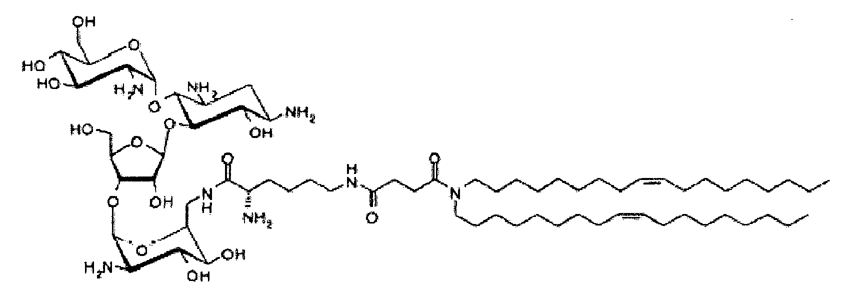
NeodiSucDODA de formule : 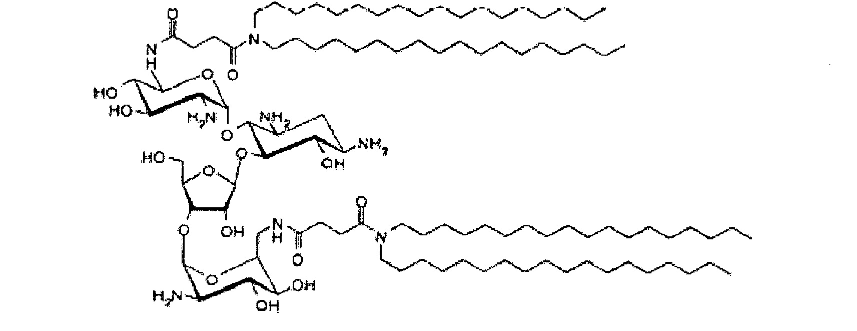
NeodiLysSucDOLA de formule : 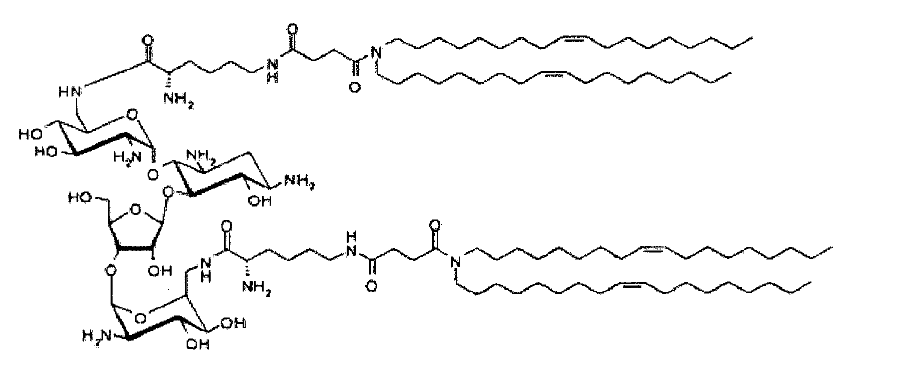
et [ParomoLys]₂-Glu-Lys-[SucDOLA]₂ de formule :

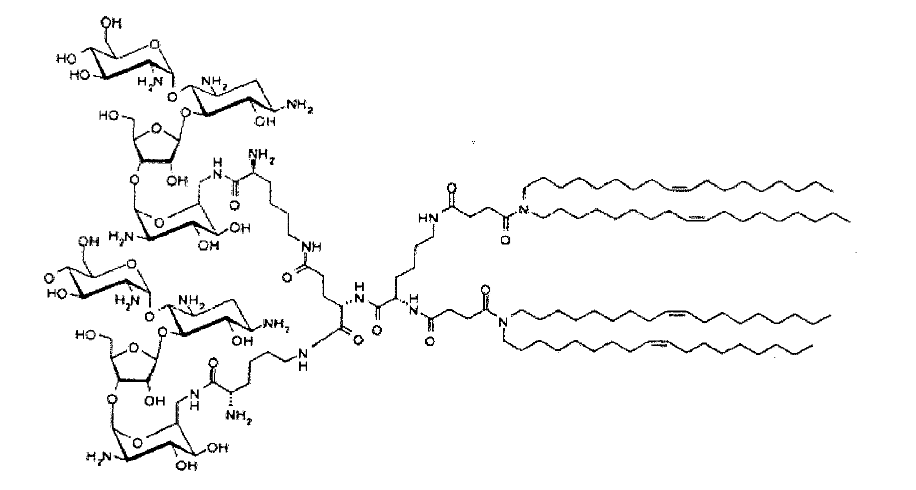

7. Composition selon l'une quelconque des revendications 1 à 6, comprenant en outre au moins un adjuvant, de préférence au moins l'un d'un lipide, d'un peptide, d'une protéine, et d'un polymère.

8. Composition selon la revendication 7, dans laquelle ledit au moins un adjuvant est choisi parmi des lipides neutres, choisi de préférence parmi des lipides naturels et synthétiques qui sont zwittérioniques ou manquent de charge ionique dans des conditions physiologiques.

9. Composition selon la revendication 8, dans laquelle ledit lipide neutre est choisi parmi la dioléoylphosphatidyléthanolamine, l'oléoylpalmitoylphosphatidyléthanolamine, les distéaroyl-, dipalmitoyl-et dimyristoyl-phosphatidyléthanolamines et leurs dérivés qui sont N-méthylés 1 à 3 fois, des phosphatidylglycérols, des diacylglycérols, des glycosyldiacylglycérols, des cérébrosides, des sphingolipides, des asialogangliosides, la dioléoylphosphatidylcholine, et le cholestérol.

10. Composition selon l'une quelconque des revendications 1 à 9, comprenant en outre un élément de ciblage extracellulaire ou intracellulaire, choisi de préférence parmi des sucres, des peptides, des protéines, des oligonucléotides, des lipides, des neuromédiateurs, des hormones, des vitamines, et leurs dérivés.

11. Composition selon la revendication 10, dans laquelle ledit élément de ciblage est fixé de manière covalente soit au composé de transfection selon la revendication 1, soit au siARN.

12. Composition selon l'une quelconque des revendications 1 à 11, comprenant en outre un véhicule qui est pharmaceutiquement acceptable pour une formulation injectable.

13. Composition selon l'une quelconque des revendications 1 à 11, comprenant en outre un véhicule qui est pharmaceutiquement acceptables pour une administration sur la peau et/ou les membranes muqueuses.

14. Composition selon l'une quelconque des revendications 1 à 13, pour une utilisation en tant que médicament.

15. Procédé de transfert d'un siARN dans des cellules *in vitro,* comprenant :
(1) la fourniture d'une composition selon l'une quelconque des revendications 1 à 13, et
(2) la mise en contact des cellules avec ladite composition.
